(19) Europäisches Patentamt
European Patent Office
Office européen des brevets

(11) EP 3 369 810 A1

(12) **EUROPEAN PATENT APPLICATION**
published in accordance with Art. 153(4) EPC

(43) Date of publication:
**05.09.2018 Bulletin 2018/36**

(21) Application number: **16859885.2**

(22) Date of filing: **27.10.2016**

(51) Int Cl.:
***C12N 5/0775*** *(2010.01)* ***A61K 35/28*** *(2015.01)*
***A61P 1/04*** *(2006.01)* ***A61P 1/16*** *(2006.01)*
***A61P 9/10*** *(2006.01)* ***A61P 17/06*** *(2006.01)*
***A61P 37/02*** *(2006.01)*

(86) International application number:
**PCT/JP2016/081860**

(87) International publication number:
**WO 2017/073656 (04.05.2017 Gazette 2017/18)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **27.10.2015 JP 2015211051**

(71) Applicants:
- **Kaneka Corporation**
**Osaka-shi, Osaka 530-8288 (JP)**
- **National Cerebral and Cardiovascular Center**
**Suita-shi, Osaka 565-8565 (JP)**

(72) Inventors:
- **YAMAHARA, Kenichi**
**Suita-shi**
**Osaka 565-8565 (JP)**
- **KITA, Yuta**
**Kobe-shi**
**Hyogo 650-0047 (JP)**
- **INO, Keita**
**Kobe-shi**
**Hyogo 650-0047 (JP)**
- **KOBAYASHI, Akira**
**Kobe-shi**
**Hyogo 650-0047 (JP)**

(74) Representative: **Hoffmann Eitle**
**Patent- und Rechtsanwälte PartmbB**
**Arabellastraße 30**
**81925 München (DE)**

# (54) METHOD FOR PRODUCING CELL POPULATION THAT INCLUDES MESENCHYMAL STEM CELLS, MESENCHYMAL STEM CELLS, CELL POPULATION, AND PHARMACEUTICAL COMPOSITION

(57) The object of the present invention is to provide a method for producing a cell population comprising mesenchymal stem cells having a high specific growth rate which are useful for promptly producing large amounts of cell formulations. The present invention provides a method for producing a cell population comprising mesenchymal stem cells, comprising: a step of treating a cell population comprising mesenchymal stem cells having different proliferative ability by physical stimulation or chemical stimulation, so as to select mesenchymal stem cells having relatively high proliferative ability, wherein the selected mesenchymal stem cells having relatively high proliferative ability are negative for CD106, and the expression level of a metallothionein family gene is increased in comparison to the expression level thereof in the cell population before the treatment by the physical stimulation or chemical stimulation.

EP 3 369 810 A1

**Description**

Technical Field

The present invention relates to a method for producing a cell population comprising mesenchymal stem cells (MSCs), which comprises preparing mesenchymal stem cells (MSCs) that are suitable for applied use in cell therapy. The present invention further relates to mesenchymal stem cells, a cell population comprising such mesenchymal stem cells (mesenchymal stem cell population), and a pharmaceutical composition comprising the aforementioned mesenchymal stem cells or the aforementioned cell population.

Background Art

Mesenchymal stem cells, which are also referred to as mesenchymal stromal cells, are somatic stem cells that have been reported to exist in the bone marrow, adipose tissues, etc., and such mesenchymal stem cells are capable of differentiating into bones, cartilage, and fats. Mesenchymal stem cells have been gaining attention as a potential cell source in cell therapy. Recently, it has been revealed that they also exist in the fetal appendage including the placenta, umbilical cord, and fetal membrane.

At present, mesenchymal stem cells have been gaining attention because of immunosuppressive capacity as well as differentiation capacity. Practical realization of such mesenchymal stem cells has been promoted to treat acute graft-versus-host disease (GVHD), and Crohn's disease, which is an inflammatory bowel disease, with the use of bone-marrow-derived mesenchymal stem cells. A variety of cells have been known as mesenchymal stem cells, and among others, amniotic mesenchymal stem cells have high immunosuppressive effects. Moreover, since the amnion used as a cell source can be non-invasively collected, it is expected that such amniotic mesenchymal stem cells will be applied to cell therapy that targets various immune-related diseases (Patent Document 1).

Patent Document 1 discloses a method for producing an amniotic mesenchymal cell composition, a method for cryopreserving the same, and a therapeutic agent. In addition, Patent Document 2 discloses a method for producing a sheet-like cell culture having high ability to produce cytokines, and it describes that the ability of the sheet-like cell culture to produce cytokines can be enhanced by establishing a step of freezing and thawing cells upon production of the sheet-like cell culture. Moreover, Patent Document 3 discloses a method for selecting pluripotent mesenchymal stem cells having high proliferative ability (subculture number, colony formation efficiency) from pluripotent mesenchymal stem cells, using flow cytometry. Furthermore, Patent Document 4 discloses a method for preparing an amniotic mesenchymal stem cell population, comprising (A) a step of collecting a cell population of mesenchymal cells from the amnion of a mammal, (B) a step of fractionating cells selected from the cell population of mesenchymal cells by (b1) detecting forward scattered light and side scattered light using a flow cytometer and then producing a two-dimensional distribution diagram and by (b2) establishing a square three-division gate on the two-dimensional distribution diagram, and (C) a step of sub-culturing the fractionated cells.

Prior Art Documents

Patent Documents

Patent Document 1: JP 2015-61520 A
Patent Document 2: JP 2015-15963 A
Patent Document 3: JP 2014-501110 A
Patent Document 4: International Publication WO 2013/077428

Summary of Invention

Object to be Solved by the Invention

As a result of preliminary studies conducted by the present inventors, it was found that it is not necessarily easy to promptly prepare and/or produce large amounts of mesenchymal stem cells necessary for producing cell formulations, since the mesenchymal stem cells have low proliferative ability (low specific growth rate). However, Patent Document 1 does not describe at all that specific mesenchymal stem cells having excellent characteristics are selectively prepared from mesenchymal stem cells, specifically, that mesenchymal stem cells having a high specific growth rate, which are useful for prompt production of large amounts of cell formulations, are obtained. Also, Patent Documents 2 and 3 do not describe at all that mesenchymal stem cells having a high specific growth rate, which are useful for prompt production of large amounts of cell formulations, are obtained. Patent Document 4 describes that an amniotic mesenchymal stem cell population having high proliferative ability and differentiation ability is prepared from a cell population comprising amniotic mesenchymal cells. However, the method described in Patent Document 4 is a method of fractionating cells by flow cytometry. That is, Patent Document 4 neither describes nor suggests that an amniotic mesenchymal stem cell population includes a cell population having large major axis and minor axis and a cell population having small major axis and minor axis, and that the above-described two types of cells are different from each other, in terms of resistance to physical stimulation or chemical stimulation.

As described above, to date, an attempt has been made to obtain cells, which are excellent in terms of cytokine-producing ability, passage number, and colony formation efficiency, from a specific cell population. However, an idea or an attempt has not yet been made, so far, to promptly produce large amounts of mesenchymal stem cells having a high specific growth rate, which are useful for prompt production of large amounts of cell formulations, from mesenchymal stem cells, by treating the mesenchymal stem cells by physical stimulation or chemical stimulation.

The object of the present invention is to provide a method for producing a cell population comprising mesenchymal stem cells (MSCs) having a high specific growth rate, the above-described mesenchymal stem cells, a cell population comprising the above-described mesenchymal stem cells (mesenchymal stem cell population), and a pharmaceutical composition comprising the above-described mesenchymal stem cells or the above-described cell population, all of which are useful for promptly producing large amounts of cell formulations.

Means for Solving the Object

The present inventors found: that mesenchymal stem cells include two or more types of cells having different growth rates; that these cells are different from one another in terms of resistance to physical stimulation or chemical stimulation; and that a cell population comprising the above two or more types of mesenchymal stem cells is treated by physical stimulation or chemical stimulation, so that mesenchymal stem cells having a low growth rate can be weeded out, in other words, such mesenchymal stem cells having a low growth rate can be killed, or the proliferative ability (specific growth rate) thereof can be significantly reduced (namely, the cells are hardly allowed to grow). Furthermore, the present inventors specified that mesenchymal stem cells having high proliferative ability are characterized to be negative for CD106, and that the expression level of a metallothionein family gene is increased therein.

Based on these findings, the present inventors found: that viable mesenchymal stem cells having relatively high proliferative ability can be selected and/or obtained by treating a cell population comprising mesenchymal stem cells having different proliferative ability by physical stimulation or chemical stimulation; and that the mesenchymal stem cells having relatively high proliferative ability are characterized in that they are negative for CD106, and in that the expression level of a metallothionein family gene is increased therein, or the content of such mesenchymal stem cells having relatively high proliferative ability can be increased. Moreover, the inventors also found: that a cell population comprising large quantities of such mesenchymal stem cells having relatively high proliferative ability has a high specific growth rate, and thus, such a cell population is extremely preferable for prompt production of large amounts of cells; and that large amounts of cell formulations can be promptly produced by culturing and/or amplifying these cells (cell population).

The aforementioned viewpoints and ideas, and also the aforementioned findings have led to the completion of the present invention.

Specifically, according to the present invention, the following are provided.

[1] A method for producing a cell population comprising mesenchymal stem cells, comprising:

a step of treating a cell population comprising mesenchymal stem cells having different proliferative ability by physical stimulation or chemical stimulation, so as to select mesenchymal stem cells having relatively high proliferative ability, wherein
the selected mesenchymal stem cells having relatively high proliferative ability are negative for CD106, and the expression level of a metallothionein family gene is increased in comparison to the expression level thereof in the cell population before the treatment by the physical stimulation or chemical stimulation.

[2] The method for producing a cell population according to [1], wherein the treatment of the cell population comprising mesenchymal stem cells having different proliferative ability by physical stimulation is freezing and subsequent thawing of the cell population.

[3] The method for producing a cell population according to [1] or [2], wherein the treatment of the cell population comprising mesenchymal stem cells having different proliferative ability by physical stimulation comprises a step of suspending the cell population in a cryopreservation solution, then freezing the obtained suspension, then maintaining the frozen state, and then thawing it.

[4] The method for producing a cell population according to [3], wherein the frozen state is maintained for 2 days or more.

[5] The method for producing a cell population according to any one of [1] to [4], which further comprises a step of culturing and/or sub-culturing the cell population comprising the mesenchymal stem cells obtained in the selection step.

[6] The method for producing a cell population according to any one of [1] to [5], which further comprises a cell population-obtaining step of subjecting a fetal appendage to an enzyme treatment, so as to obtain a cell population comprising mesenchymal stem cells having different proliferative ability.

[7] The method for producing a cell population according to [6], which further comprises a step of culturing the cell population obtained in the cell population-obtaining step.

[8] The method for producing a cell population according to any one of [1] to [7], wherein the metallothionein family gene is at least one selected from the group consisting of MT1E, MT1F, MT1G, MT1H, MT1X, and MT2A.

[9] The method for producing a cell population according to any one of [1] to [8], wherein the selected mesenchymal stem cells having relatively high proliferative ability are negative for SA-β-Gal.

[10] The method for producing a cell population according to [9], wherein the average rate of the SA-β-Gal-negative mesenchymal stem cells in the cell population is 90% or more.

[11] The method for producing a cell population according to any one of [1] to [10], wherein the selected mesenchymal stem cells having relatively high proliferative ability is positive for CD105, CD73, and CD90, and negative for CD45, CD34, CD11b, CD79alpha, and HLA-DR.

[12] The method for producing a cell population according to any one of [1] to [11], wherein the specific growth rate of the selected mesenchymal stem cells having relatively high proliferative ability is 0.14 (1/day) or more.

[13] The method for producing a cell population according to any one of [1] to [11], wherein the average diameter of the selected mesenchymal stem cells having relatively high proliferative ability that are in a floating state is 80% or less of the average diameter of mesenchymal stem cells having relatively low proliferative ability that are in a floating state.

[14] Mesenchymal stem cells, which are negative for CD106, and wherein the expression level of a metallothionein family gene is increased in comparison to the expression level thereof in the cells before the treatment by the physical stimulation or chemical stimulation.

[15] The mesenchymal stem cells according to [14], wherein the metallothionein family gene is at least one selected from the group consisting of MT1E, MT1F, MT1G, MT1H, MT1X, and MT2A.

[16] The mesenchymal stem cells according to [14] or [15], wherein the mesenchymal stem cells are negative for SA-β-Gal.

[17] The mesenchymal stem cells according to any one of [14] to [16], wherein the mesenchymal stem cells are positive for CD105, CD73, and CD90, and negative for CD45, CD34, CD11b, CD79alpha, and HLA-DR.

[18] The mesenchymal stem cells according to any one of [14] to [17], wherein the specific growth rate of the mesenchymal stem cells is 0.14 (1/day) or more.

[19] The mesenchymal stem cells according to any one of [14] to [18], wherein the average diameter of the mesenchymal stem cells that are in a floating state is 80% or less of the average diameter of mesenchymal stem cells having relatively low proliferative ability that are in a floating state.

[20] A cell population comprising mesenchymal stem cells, wherein
the expression level of a metallothionein family gene in the mesenchymal stem cells is increased in comparison to the expression level thereof in the cells before the treatment by the physical stimulation or chemical stimulation, and the mesenchymal stem cells are negative for CD106.

[21] The cell population according to [20], wherein the rate of the CD106-positive mesenchymal stem cells in the cell population is less than 5%.

[22] The cell population according to [20] or [21], wherein the average rate of the SA-β-Gal-negative mesenchymal stem cells in the cell population is 90% or more.

[23] A pharmaceutical composition comprising the mesenchymal stem cells according to any one of [14] to [19], or the cell population according to any one of [20] to [22], and a pharmaceutically acceptable medium.

[24] The pharmaceutical composition according to [23], wherein the single dose of the mesenchymal stem cells to a human is $10^9$ cells/kg body weight or less.

[25] The pharmaceutical composition according to [23] or [24], which is a therapeutic agent for a disease selected from immune-related disease, graft-versus-host disease, inflammatory bowel disease, systemic lupus erythematosus, connective tissue disease, radiation enteritis, hepatic cirrhosis, stroke, or atopic dermatitis.

[26] Mesenchymal stem cells obtained by the production method according to any one of [1] to [13].

[27] Use of the mesenchymal stem cells according to any one of [14] to [19] or the cell population according to any one of [20] to [22] for the production of a pharmaceutical composition.

[28] The use according to [27], wherein the pharmaceutical composition comprises mesenchymal stem cells, the single dose of which to a human is $10^9$ cells/kg body weight or less.

[29] The use according to [27] or [28], wherein the pharmaceutical composition is a therapeutic agent for a disease selected from immune-related disease, graft-versus-host disease, inflammatory bowel disease, systemic lupus erythematosus, connective tissue disease, radiation enteritis, hepatic cirrhosis, stroke, or atopic dermatitis.

[30] The mesenchymal stem cells according to any one of [14] to [19], or the cell population according to any one of [20] to [22], for use in the treatment of a disease.

[31] The mesenchymal stem cells or the cell population according to [30], wherein the single dose of the mesenchymal stem cells to a human is $10^9$ cells/kg body weight or less.

[32] The mesenchymal stem cells or the cell population according to [30] or [31], wherein the disease is selected from immune-related disease, graft-versus-host disease, inflammatory bowel disease, systemic lupus erythematosus, connective tissue disease, radiation enteritis, hepatic cirrhosis, stroke, or atopic dermatitis.

[33] A method for treating a disease, comprising administering the mesenchymal stem cells according to any one of [14] to [19], or the cell population according to any one of [20] to [22], to a patient in need of therapy.

[34] The method for treating a disease according to [33], wherein the single dose of the mesenchymal stem cells to a human is $10^9$ cells/kg body weight or less.

[35] The method for treating a disease according to [33] or [34], wherein the disease is selected from immune-related disease, graft-versus-host disease, inflammatory bowel disease, systemic lupus erythematosus, connective tissue disease, radiation enteritis, hepatic cirrhosis, stroke, or atopic dermatitis.

[36] A composition comprising the mesenchymal stem cells according to any one of [14] to [19] and a medium.

[37] A composition comprising the cell population according to any one of [20] to [22] and a medium.

Advantageous Effects of Invention

According to the present invention, mesenchymal stem cells (cell population) with a high specific growth rate can be obtained, and thereby, a cell formulation (pharmaceutical composition) can be promptly produced in a large amount. For example, the cells can be prepared and/or produced in an amount of $2.3 \times 10^3$ (cells/cm$^2$/day) or more per batch culture. Moreover, according to the present invention, mesenchymal stem cells (cell population) with improved resistance to freezing and thawing can be obtained.

Brief Description of Drawings

[Fig.1] Fig. 1 shows a trypan blue staining image of amniotic MSCs in a floating state, immediately after completion of the freezing-thawing treatment in Step 1-5 in Example 1.

[Fig.2] Fig. 2 shows a phase-contrast microscopic image of the cells of pre-culture 2 (amniotic MSCs before being subjected to the freezing-thawing treatment) obtained in Step 1-4 in Example 1, which were in a confluent state.

[Fig.3] Fig. 3 shows a phase-contrast microscopic image of the cells of main culture 1 (amniotic MSCs that were cultured once after completion of the freezing-thawing treatment) obtained in Step 1-6 in Example 1, which were in a confluent state.

[Fig.4] Fig. 4 shows a phase-contrast microscopic image of the cells of subculture 1 (amniotic MSCs that were subcultured once after completion of the freezing-thawing treatment) obtained in Step 1-6 in Example 1, which were in a confluent state.

[Fig.5] Fig. 5 shows a SA-β-Gal staining image of the cells of pre-culture 2 (amniotic MSCs before being subjected to the freezing-thawing treatment) obtained in Step 1-4 in Example 1.

[Fig.6] Fig. 6 shows a SA-β-Gal staining image of the cells of pre-culture 3 (amniotic MSCs before being subjected to the freezing-thawing treatment) obtained in Step 3-5 in Example 3.

[Fig.7] Fig. 7 shows a SA-β-Gal staining image of the cells of subculture 2 (amniotic MSCs that were subcultured twice after completion of the freezing-thawing treatment) obtained in Step 3-9 in Example 3.

Embodiment of Carrying out the Invention

Embodiments of the present invention are specifically explained below. However, the below-mentioned explanations are intended to facilitate understanding of the present invention, and therefore, the scope of the present invention is not limited to the following embodiments. The present invention encompasses other embodiments with appropriate modifications made by a person skilled in the art.

[1] Explanation of terms

The term "fetal appendage" used herein refers to a fetal membrane, a placenta, an umbilical cord, and amniotic fluid. In addition, the term "fetal membrane" refers to a fetal sac containing fetal amniotic fluid, which comprises an amnion, a chorion, and a decidua in that order from the inside. The amnion and chorion are originated from the fetus. The term "amnion" refers to a transparent thin membrane with few blood vessels, which is located in the most inner layer of the fetal membrane. The inner layer of the amnion (also referred to as an "epithelial cell layer") is covered with a layer of epithelial cells having a secretory function and secretes amniotic fluid. The outer layer of the amnion (also referred to as an "extracellular matrix layer," corresponding to the interstitial) comprises mesenchymal stem cells.
The term "mesenchymal stromal cells (MSCs)" used in the present description refers to stem cells, which satisfy the below-mentioned definition and which are used without being distinguished from "mesenchymal stromal cells." In the present description, the "mesenchymal stem cells" may also be referred to as "MSCs."
Definition of mesenchymal stem cells

i) Adherence to plastic in standard medium under culture conditions
ii) Specific surface antigen expression (positive for CD105, CD73, and CD90, and negative for CD45, CD34, CD14, CD11b, CD79alpha, CD19, and HLA-DR)

The term "amniotic mesenchymal stem cells" used in the present description refers to mesenchymal stem cells derived from the amnion, and this term is used without being distinguished from "amniotic mesenchymal stromal cells." In the present description, the "amniotic mesenchymal stem cells" may also be referred to as "amniotic MSCs."
The "mesenchymal stem cell population" used in the present description refers to a cell population comprising mesenchymal stem cells. The form of this cell population is not particularly limited, and examples thereof include a cell pellet, a cell aggregate, a cell floating solution, and a cell suspension.
The term "cell population comprising mesenchymal stem cells having different proliferative ability" means a cell population comprising, at least, mesenchymal stem cells having relatively high proliferative ability and mesenchymal stem cells having relatively low proliferative ability. The present inventors found that the above-described mesenchymal stem cells having relatively low proliferative ability have relatively low resistance to physical stimulation or chemical stimulation, whereas the above-described mesenchymal stem cells having relatively high proliferative ability have relatively high resistance to physical stimulation or chemical stimulation. In a case where there are two types of mesenchymal stem cells each having different proliferative ability, cells having relatively high proliferative ability are referred to as "mesenchymal stem cells having relatively high proliferative ability," whereas cells having relatively low proliferative ability are referred to as "mesenchymal stem cells having relatively low proliferative ability."
The term "cell count obtained per batch culture" used in the present description means a cell count obtained per unit surface area of a culture vessel and per unit culture day, in a single culture. Accordingly, the unit of the "cell count obtained per batch culture" is (cells/$cm^2$/day).
The term "relatively" used in the present description means a difference (high and low, or large and small) in a case where a subject is compared with a counterpart. For example, when a cell population comprising mesenchymal stem cells having different proliferative ability is classified into mesenchymal stem cells having high proliferative ability and mesenchymal stem cells having low proliferative ability, using proliferative ability as an indicator, the former mesenchymal stem cells having high proliferative ability are referred to as "mesenchymal stem cells having relatively high proliferative ability," and the latter mesenchymal stem cells having low proliferative ability are referred to as "mesenchymal stem cells having relatively low proliferative ability."
Similarly, when a cell population comprising cells having different sizes is classified into cells having a large size and cells having a small size, using size as an indicator, the former cells having a large size are referred to as "cells having a relatively large size," and the latter cells having a small size are referred to as "cells having a relatively small size."
The term "proliferative ability" used in the present description means the ability of cells to increase a cell count thereof as a result of cell division. The proliferative ability of mesenchymal stem cells (MSCs) in a mesenchymal stem cell population can be evaluated using a specific growth rate. The method of measuring a specific growth rate is as described later in the present description.

[2] Method for producing a cell population comprising mesenchymal stem cells

The method for producing a cell population comprising mesenchymal stem cells according to the present invention is a method comprising a selection step of selecting mesenchymal stem cells having relatively high proliferative ability from a cell population comprising mesenchymal stem cells (MSCs) having different proliferative ability, by treating the cell population by physical stimulation or chemical stimulation.
The phrase "to select mesenchymal stem cells having relatively high proliferative ability" is intended to mean a state in which the rate of mesenchymal stem cells having relatively high proliferative ability in a cell population comprising mesenchymal stem cells is increased by weeding out mesenchymal stem cells having a relatively low growth rate, namely, by killing such mesenchymal stem cells having a relatively low growth rate or by significantly reducing the proliferative ability (specific growth rate) thereof. However, the state expressed by the above phrase is not limited thereto.

The cell population comprising MSCs having different proliferative ability is a cell population comprising, at least, MSCs having relatively high proliferative ability and MSCs having relatively low proliferative ability.

The production method of the present invention may comprise a cell population-obtaining step of obtaining a cell population comprising MSCs having different proliferative ability by performing an enzyme treatment on a sample (e.g., a fetal appendage such as amnion, etc.).

The amnion comprises an epithelial cell layer and an extracellular matrix layer, and the latter layer comprises amniotic MSCs. Amniotic epithelial cells are characterized in that they express epithelial cadherin (E-cadherin: CD324) and an epithelial cell adhesion factor (EpCAM: CD326), as with other epithelial cells. On the other hand, amniotic MSCs do not express such epithelium-specific surface antigen markers. Thus, the two types of cells can be easily distinguished from each other by flow cytometry. The above-described cell population-obtaining step may also be a step of obtaining the amnion by Caesarean section.

The enzyme treatment performed on a sample (e.g., a fetal appendage such as amnion, etc.) is preferably a treatment of using an enzyme (or a combination of enzymes), which can release MSCs comprised in the extracellular matrix layer of the sample, and does not decompose the epithelial cell layer of the sample. The type of such enzyme is not particularly limited. Examples of the enzyme include collagenase and/or metalloproteinase. Examples of the metalloproteinase include thermolysin and/or dispase, which are metalloproteinases cleaving the N-terminal side of a non-polar amino acid, but are not limited thereto.

The concentration of collagenase is preferably 50 CDU/ml or more, more preferably 75 CDU/ml or more, even more preferably 100 CDU/ml or more, further preferably 125 CDU/ml or more, and still further preferably 150 CDU/ml or more. On the other hand, although the concentration of collagenase is not particularly limited, it is for example, 1000 CDU/ml or less, 900 CDU/ml or less, 800 CDU/ml or less, 700 CDU/ml or less, 600 CDU/ml or less, 500 CDU/ml or less, 400 CDU/ml or less, or 300 CDU/ml or less. Herein, CDU (collagen digestion unit) is defined to be the amount of enzyme necessary for generating amino acids and peptides corresponding to 1 μmol of leucine at 37°C at pH 7.5 for 5 hours, using collagen as a substrate. The concentration of metalloproteinase (e.g., thermolysin and/or dispase) is preferably 100 PU/ml or more, more preferably 125 PU/ml or more, even more preferably 150 PU/ml or more, further preferably 175 PU/ml or more, and still further preferably 200 PU/ml or more. On the other hand, the concentration of metalloproteinase is preferably 800 PU/ml or less, more preferably 700 PU/ml or less, even more preferably 600 PU/ml or less, further preferably 500 PU/ml or less, and still further preferably 400 PU/ml or less. Herein, PU (protease unit) is defined to be the amount of enzyme necessary for generating amino acids and peptides corresponding to 1 μg of tyrosine at 35°C at pH 7.2 for 1 minute, using lactic acid casein as a substrate. In the above-described enzyme concentration range, MSCs comprised in the extracellular matrix layer of a sample can be efficiently released, while preventing the mixing of epithelial cells comprised in the epithelial cell layer thereof. If the concentration of collagenase is set at less than 50 CDU/ml, or if the concentration of metalloproteinase is set at less than 100 CDU/ml, there may be a case where digestion of the extracellular matrix layer becomes insufficient and the recovery rate of MSCs is significantly reduced. On the other hand, if the concentration of metalloproteinase is set at greater than 800 CDU/ml, since epithelial cells are mixed into a digestive fluid together with decomposition of the epithelial cell layer, there may be case where the recovery rate of MSCs is relatively reduced. A combination of the preferred concentrations of collagenase and/or metalloproteinase can be determined by microscopic observation of the sample after completion of the enzyme treatment, or flow cytometry performed on the obtained cells. In addition, MSCs comprising almost no epithelial cells are preferably treated by the after-mentioned physical stimulation or chemical stimulation.

From the viewpoint of the efficient recovery of viable cells, it is preferable to treat the sample, simultaneously and collectively, by the combination of collagenase and metalloproteinase. In this case, thermolysin and/or dispase can be used as metalloproteinases, but usable enzymes are not limited thereto. MSCs can be simply obtained by treating the sample only once with an enzyme solution containing collagenase and metalloproteinase. Moreover, by treating the sample simultaneously and collectively, contamination risk with bacteria, viruses, etc. can be reduced.

With regard to the enzyme treatment of a sample, it is preferable that a sample, which has been washed with a washing solution such as a normal saline or a Hanks' balanced salt solution, be immersed in an enzyme solution, and be then treated, while being stirred by a stirring means. From the viewpoint of the efficient release of MSCs comprised in the extracellular matrix layer of a sample, for example, a stirrer or a shaker can be used as such a stirring means, but examples of the stirring means are not limited thereto. The stirring rate is not particularly limited. When a stirrer or a shaker is used, the stirring rate is, for example, 5 rpm or more, 10 rpm or more, 20 rpm or more, 30 rpm or more, 40 rpm or more, or 50 rpm or more. In addition, although such a stirring rate is not particularly limited, when a stirrer or a shaker is used, it is, for example, 100 rpm or less, 90 rpm or less, 80 rpm or less, 70 rpm or less, or 60 rpm or less. The time required for the enzyme treatment is not particularly limited, and it is, for example, 10 minutes or more, 20 minutes or more, 30 minutes or more, 40 minutes or more, 50 minutes or more, or 60 minutes or more. In addition, although the time required for the enzyme treatment is not particularly limited, and it is, for example, 6 hours or less, 5 hours or less, 4 hours or less, 3 hours or less, 2 hours or less, 110 minutes or less, 100 minutes or less, 90 minutes or less, 80 minutes or less, or 70 minutes or less. The temperature required for the enzyme treatment is not particularly limited, and it is, for example, 15°C or higher, 16°C or higher, 17°C or higher, 18°C or higher, 19°C or higher, 20°C or higher, 21°C or higher, 22°C or higher, 23°C or higher, 24°C or higher, 25°C or higher, 26°C or higher, 27°C or higher, 28°C or higher, 29°C or higher, 30°C or higher, 31°C or higher, 32°C or higher, 33°C or higher, 34°C or higher, 35°C or higher, or 36°C or higher. In addition, the temperature required for the enzyme treatment is not particularly limited, and it is, for example, 40°C or lower, 39°C or lower, 38°C or lower, or 37°C or lower.

In the production method of the present invention, an enzyme solution comprising the released MSCs can be filtered through a filter, as desired. Since only the released cells are passed through a filter and an undecomposed epithelial cell layer cannot be passed through the filter and remains on the filter, the released MSCs can be easily recovered, and further, contamination risk with bacteria, viruses, etc. can be reduced. The filter used herein is not particularly limited, and it is, for example, a mesh filter. The pore size (the size of mesh) of such a mesh filter is not particularly limited, and it is, for example, 40 μm or more, 50 μm or more, 60 μm or more, 70 μm or more, 80 μm or more, 90 μm or more, 100 μm or more, 110 μm or more, 120 μm or more, 130 μm or more, or 140 μm or more. In addition, the pore size of such a mesh filter is not particularly limited, and it is, for example, 200 μm or less, 190 μm or less, 180 μm or less, 170 μm or less, 160 μm or less, or 150 μm or less. The filtration rate is not particularly limited. By setting the pore size of a mesh filter to be within the above-described range, an enzyme solution comprising MSCs can be filtered by a free fall motion, and thereby, a reduction in cell survival rate can be prevented.

Regarding mesh material, a nylon mesh is preferably used. A tube having a 40 μm, 70 μm, or 100 μm nylon mesh filter, such as a Falcon cell strainer that is widely used for research purposes is available. Alternatively, a medical mesh cloth (nylon and polyester) used for hemodialysis and the like is available. Further, an arterial filter used for extracorporeal circulation (a polyester mesh filter; pore size: 40 μm or more and 120 μm or less) is also available. A mesh made of other material such as a stainless-steel mesh filter can also be used.

Preferably, MSCs are allowed to pass through a mesh in a free fall motion. It is also possible to force the cells to pass through a mesh by suction using a pump or the like. In this case, however, in order to avoid damage of cells, minimum necessary pressurization is desirable.

MSCs that have passed through a filter are diluted with two times or more its volume of a medium or balanced salt buffer solution. Thereafter, MSCs can be recovered by centrifugation. Examples of the balanced salt buffer solution that can be used herein include buffered solutions such as Dulbecco's phosphate-buffered saline (DPBS), Earle's balanced salt solution (EBSS), Hank's balanced salt solution (HBSS), and phosphate-buffered saline (PBS), but are not limited thereto.

The cell population obtained in the cell population-obtaining step can be allowed to proliferate by culture, before being subjected to the above-described selection step, as desired. That is to say, the production method of the present invention may further comprise a step of culturing the cell population obtained in the cell population-obtaining step. Such culture (pre-culture) is preferably carried out in a plastic dish or a flask in an environment of a $CO_2$ concentration of 3% or more and 5% or less and 37°C. In addition, in the step of culturing the cell population obtained in the cell population-obtaining step, the cells may be subcultured one or more times.

The medium used in the above-described culture is not particularly limited, and examples of the medium include αMEM (Alpha Modification of Minimum Essential Medium Eagle), DMEM, BME, BGJb, CMRL1066, Glasgow MEM, Improved MEM Zinc Option, IMDM (Iscove's Modified Dulbecco's Medium), Eagle MEM, RPMI1640, M199, Ham's F10 medium, Ham's F12 medium, Fischer's medium, mixed medium (e.g., DMEM/F12 medium), serum free medium, and a medium comprising the aforementioned medium as a basal medium. Examples of the serum free medium include STK1, STK2 (DS Pharma Biomedical Co., Ltd.), EXPREP MSCs Medium (BioMimetics Sympathies), and Corning Stemgro human mesenchymal stem cell medium (Corning), but examples thereof are not particularly limited. To these media, other components, such as albumin, serum, a serum replacement reagent, a growth factor, and a growth factor-stabilizing reagent (such as heparin), may be added, as necessary, but such other components are not particularly limited thereto. In the case of albumin, the concentration thereof is preferably more than 0.05% and 5% or less. In the case of serum, the concentration is preferably 5% or more.

The production method of the present invention comprises a selection step of selecting MSCs having relatively high proliferative ability by treating a cell population comprising MSCs having different proliferative ability by physical stimulation or chemical stimulation (preferably, physical stimulation).

The type of such physical stimulation or chemical stimulation is not particularly limited, as long as it is able to separate MSCs having relatively high proliferative ability from MSCs having relatively low proliferative ability. An example of such stimulation is stimulation, in which the resistance of MSCs having relatively high proliferative ability to the stimulation is higher than the resistance of MSCs having relatively low proliferative ability to the stimulation, but the example of the stimulation is not limited thereto.

Examples of the physical stimulation include, but are not particularly limited to, freezing and thawing, heating, filtration, centrifugation, electrophoresis, voltage application, pressurization, decompression, osmotic changes, ultraviolet irradiation, and ultrasonic irradiation. A plurality of physical stimulations can be applied singly or in combination, but application of the physical stimulation is not limited thereto.

Examples of the chemical stimulation include, but are not particularly limited to, gene transfer, a treatment with cytotoxic compounds, and a pH change treatment with an acid or a base. A plurality of chemical stimulations can be applied singly or in combination, but application of the physical stimulation is not limited thereto.

A preferred example of the physical stimulation is the freezing of a cell population comprising MSCs having different proliferative ability and the subsequent thawing thereof. A more preferred example of the physical stimulation is that a cell population comprising MSCs having different proliferative ability is suspended in a cryopreservation solution, the obtained suspension is then frozen, the frozen state is then maintained, and it is then thawed.

A preferred example of the chemical stimulation is that a metallothionein family gene is introduced into a cell population comprising CD106-negative MSCs, using a vector comprising the nucleotide sequence of the metallothionein family gene. A more specific aspect of the gene transfer is gene transfer using a viral vector (e.g., a retrovirus vector, a lentivirus vector, an adenovirus vector, an adeno-associated virus vector, etc.), or a non-viral vector (e.g., plasmid DNA, an artificial chromosome vector, etc.), but the gene transfer is not particularly limited thereto.

From the viewpoint of enhancing the survival rate of MSCs having relatively high proliferative ability, the above-described cryopreservation solution preferably comprises a predetermined concentration of polysaccharide. The preferred concentration of such a polysaccharide is, for example, 1% by mass or more, 2% by mass or more, 3% by mass or more, 4% by mass or more, 5% by mass or more, 6% by mass or more, 7% by mass or more, 8% by mass or more, 9% by mass or more, 10% by mass or more, 11% by mass or more, or 12% by mass or more. On the other hand, the preferred concentration of such a polysaccharide is, for example, 40% by mass or less, 35% by mass or less, 30% by mass or less, 25% by mass or less, 20% by mass or less, 19% by mass or less, 18% by mass or less, 17% by mass or less, 16% by mass or less, 15% by mass or less, 14% by mass or less, or 13% by mass or less. Examples of the polysaccharide include, but are not limited to, hydroxyethyl starch (HES) and dextran (Dextran 40, etc.).

From the viewpoint of efficiently selecting MSCs having relatively high proliferative ability, the above-described cryopreservation solution preferably comprises a predetermined concentration of dimethyl sulfoxide (DMSO). The preferred concentration of DMSO is, for example, 1% by mass or more, 2% by mass or more, 3% by mass or more, 4% by mass or more, 5% by mass or more, 6% by mass or more, 7% by mass or more, 8% by mass or more, or 9% by mass or more. On the other hand, the preferred concentration of DMSO is, for example, 20% by mass or less, 19% by mass or less, 18% by mass or less, 17% by mass or less, 16% by mass or less, 15% by mass or less, 14% by mass or less, 13% by mass or less, 12% by mass or less, 11% by mass or less, or 10% by mass or less.

From the viewpoint of enhancing the survival rate of MSCs having relatively high proliferative ability, the above-described cryopreservation solution preferably comprises a predetermined concentration of albumin, which is more than 0% by mass. The preferred concentration of albumin is, for example, 0.5% by mass or more, 1% by mass or more, 2% by mass or more, 3% by mass or more, 4% by mass or more, 5% by mass or more, 6% by mass or more, 7% by mass or more, or 8% by mass or more. On the other hand, the preferred concentration of albumin is, for example, 40% by mass or less, 35% by mass or less, 30% by mass or less, 25% by mass or less, 20% by mass or less, 15% by mass or less, 10% by mass or less, or 9% by mass or less. Examples of the albumin include, but are not limited to, bovine serum albumin, mouse albumin, and human albumin.

A means for freezing a cell population comprising MSCs having different proliferative ability is not particularly limited, and it is, for example, a program freezer, a deep freezer, and immersion in liquid nitrogen. The preferred temperature at which the cells are frozen is, for example, -30°C or lower, -40°C or lower, -50°C or lower, -60°C or lower, -70°C or lower, -80°C or lower, -90°C or lower, -100°C or lower, -110°C or lower, -120°C or lower, or -130°C or lower. On the other hand, the preferred temperature at which the cells are frozen is, for example, -196°C (the temperature of liquid nitrogen) or higher, -190°C or higher, -180°C or higher, -170°C or higher, -160°C or higher, -150°C or higher, or -140°C or higher. The preferred rate at which the cells are frozen is, for example, -1°C/min, -2°C/min, -3°C/min, -4°C/min, -5°C/min, -6°C/min, -7°C/min, -8°C/min, -9°C/min, -10°C/min, -11°C/min, -12°C/min, -13°C/min, -14°C/min, or -15°C/min. When a program freezer is used as such a freezing means, the temperature can be decreased to a temperature that is between -50°C or higher and -30°C or lower (e.g., -40°C) at a freezing rate of -2°C/min or more and -1°C/min or less, and the temperature can be further decreased to a temperature that is between -100°C or higher and -80°C or lower (e.g., -90°C) at a freezing rate of -11°C/min or more and -9°C/min or less (e.g., -10°C/min).

When the cell population is frozen by the above-described freezing means, the above-described cell population may be frozen in a state in which it is placed in any given storage vessel. Examples of such a storage vessel include a cryotube, a cryovial, a freezing bag, and an infusion bag, but are not limited thereto.

From the viewpoint of efficiently selecting MSCs having relatively high proliferative ability, the above-described frozen cell population is preferably maintained at a temperature, at which the frozen state can be maintained, for a predetermined period of time. The upper limit of the preferred temperature applied upon maintaining the frozen state is, for example, -130°C or lower, -140°C or lower, or -150°C or lower. On the other hand, the lower limit of the preferred temperature applied upon maintaining the frozen state is, for example, -196°C (the temperature of liquid nitrogen) or higher, -190°C or higher, -180°C or higher, -170°C or higher, or -160°C or higher. Since the glass transition temperature of the cytoplasm is -130°C, if the temperature applied upon maintaining the frozen state is set at higher than -130°C, there may be case where ice crystals are generated in cells and the cell survival rate is decreased. The lower limit of the preferred period of time, during which the cells are maintained in a frozen state, is for example, 12 hours or more, 16 hours or more, 20 hours or more, 24 hours or more, 30 hours or more, 36 hours or more, 42 hours or more, 2 days or more, 3 days or more, 4 days or more, 5 days or more, 6 days or more, 7 days or more, 8 days or more, 9 days or more, 10 days or more, 11 days or more, 12 days or more, 13 days or more, 14 days or more, 15 days or more, 16 days or more, 17 days or more, 18 days or more, 19 days or more, 20 days or more, 21 days or more, 22 days or more, 23 days or more, 24 days or more, 25 days or more, 26 days or more, 27 days or more, 28 days or more, 29 days or more, 30 days or more, or 31 days or more. On the other hand, the upper limit of the period of time during which the cells are maintained in a frozen state is not particularly limited, and it is, for example, 20 years or less, 10 years or less, 5 years or less, 4 years or less, 3 years or less, 2 years or less, 1 year or less, 11 months or less, 10 months or less, 9 months or less, 8 months or less, 7 months or less, 6 months or less, 5 months or less, 4 months or less, 3 months or less, 2 months or less, or 1 month or less.

A means for thawing the thus frozen cell population is, for example, the contact (e.g., immersion) of the frozen cell population with a medium (e.g., a solid, a liquid, or a gas) having a temperature higher than the freezing temperature, but is not limited thereto. The lower limit of the temperature of the medium is not particularly limited, and it is, for example, 1°C or higher, 2°C or higher, 3°C or higher, 4°C or higher, 5°C or higher, 6°C or higher, 7°C or higher, 8°C or higher, 9°C or higher, 10°C or higher, 11°C or higher, 12°C or higher, 13°C or higher, 14°C or higher, 15°C or higher, 16°C or higher, 17°C or higher, 18°C or higher, 19°C or higher, 20°C or higher, 21°C or higher, 22°C or higher, 23°C or higher, 24°C or higher, 25°C or higher, 26°C or higher, 27°C or higher, 28°C or higher, 29°C or higher, 30°C or higher, 31°C or higher, 32°C or higher, 33°C or higher, 34°C or higher, 35°C or higher, or 36°C or higher. On the other hand, the upper limit of the temperature of the medium is not particularly limited, and it is, for example, 50°C or lower, 49°C or lower, 48°C or lower, 47°C or lower, 46°C or lower, 45°C or lower, 44°C or lower, 43°C or lower, 42°C or lower, 41°C or lower, 40°C or lower, 39°C or lower, 38°C or lower, or 37°C or lower. The time required for thawing is not particularly limited, and it is, for example, 10 seconds, 20 seconds, 30 seconds, 40 seconds, 50 seconds, 60 seconds (1 minute), 70 seconds, 80 seconds, 90 seconds, 100 seconds, 110 seconds, 120 seconds (2 minutes), 3 minutes, 4 minutes, 5 minutes, 6 minutes, 7 minutes, 8 minutes, 9 minutes or 10 minutes. For example, when 1 ml of frozen-state cell suspension filled in a 2-ml cryotube made of polypropylene (outer diameter: 13.5 mm) is thawed in a thermostatic bath at 37°C, it can be thawed within 2 minutes. Moreover, for example, when 20 ml of frozen-state cell suspension filled in a 25-ml freezing bag made of polyolefin (outer size: 81 mm x 121.5 mm) is thawed in a thermostatic bath at 37°C, it can be thawed within 5 minutes. The survival rate of MSCs having relatively high proliferative ability can be improved by rapidly thawing the cells.

The cell population comprising MSCs obtained by the above-described selection step can be further cultured and/or subcultured, and thereby, large amounts of MSCs having relatively high proliferative ability (the after-mentioned MSCs having a high specific growth rate) can be obtained, and further, the content of MSCs having relatively high proliferative ability in the obtained cell population can be increased. That is to say, the production method of the present invention may be a production method further comprising a step of culturing and/or subculturing the cell population comprising MSCs obtained in the selection step. The content of the above-described MSCs having relatively high proliferative ability in the mesenchymal stem cell population obtained by performing culture and/or subculture after the above-described selection step is 10% or more, preferably 20% or more, more preferably 30% or more, even more preferably 40% or more, further preferably 50% or more, still further preferably 60% or more, still further preferably 70% or more, still further preferably 80% or more, still further preferably 90% or more, still further preferably 95% or more, still further preferably 98% or more, still further preferably 99% or more, and particularly preferably 100%. It is to be noted that the content of MSCs having relatively high proliferative ability in a cell population before a stimulation treatment is different depending on a donor. As described in the Examples later, the present inventors found that the content is generally 1% or less.

The MSCs obtained by the above-described selection step can be cultured (main culture), for example, by the following steps. First, a melted cell suspension after completion of the freezing-thawing treatment is centrifuged, the obtained supernatant is then removed, and the obtained cell pellets are suspended in a medium. Subsequently, the cells are seeded on a culture vessel made of plastic, and are then cultured in a medium in an environment of a $CO_2$ concentration of 3% or more and 5% or less and 37°C, so that the confluent percentage can be 95% or less. Examples of the above-described medium include αMEM, DMEM, BME, BGJb, CMRL1066, Glasgow MEM, Improved MEM Zinc Option, IMDM, Eagle MEM, RPMI1640, M199, Ham's F10 medium, Ham's F12 medium, Fischer's medium, mixed medium (e.g., DMEM/F12 medium), serum free medium, and a medium comprising the aforementioned medium as a basal medium, but the examples of the medium are not limited thereto. To these media, additional components, such as albumin, serum, a serum replacement reagent, a growth factor, and a growth factor-stabilizing reagent, may be added, as necessary, but such additional components are not particularly limited thereto. The cells obtained by the above-described culture (main culture) are once cultured cells.

The above-described once cultured cells can be further subcultured and cultured, for example, as follows. First, the once cultured cells are treated with ethylenediaminetetraacetic acid (EDTA), and are then treated with trypsin, so that the cells are peeled from the plastic culture vessel. Subsequently, the obtained cell suspension is centrifuged, the obtained supernatant is then removed, and the obtained cell pellets are suspended in a medium. Finally, the cells are seeded on a plastic culture vessel, and are then cultured in a medium in an environment of a $CO_2$ concentration of 3% or more and 5% or less and 37°C, so that the confluent percentage can be 95% or less. Examples of the above-described medium include αMEM, DMEM, BME, BGJb, CMRL1066, Glasgow MEM, Improved MEM Zinc Option, IMDM, Eagle MEM, RPMI1640, M199, Ham's F10 medium, Ham's F12 medium, Fischer's medium, mixed medium (e.g., DMEM/F12 medium), serum free medium, and a medium comprising the aforementioned medium as a basal medium, but the examples of the medium are not limited thereto. To these media, additional components, such as albumin, serum, a serum replacement reagent, a growth factor, and a growth factor-stabilizing reagent, may be added, as necessary, but such additional components are not particularly limited thereto. The cells obtained by the above-described subculture and culture are once subcultured cells. By performing the same subculture and culture as described above, the cells that have been subcultured an N time(s) can be obtained (wherein N indicates an integer of 1 or greater). From the viewpoint of producing large amounts of cells, the lower limit of the number of subcultures N is preferably, for example, 1 or more, 2 or more, 3 or more, 4 or more, 5 or more, 6 or more, 7 or more, 8 or more, or 9 or more. On the other hand, from the viewpoint of suppressing the aging of cells, the upper limit of the number of subcultures N is preferably, for example, 50 or less, 45 or less, 40 or less, 35 or less, 30 or less, 25 or less, or 20 or less.

The mesenchymal stem cells having relatively high proliferative ability, which are selected by the above-described method of the present invention, are characterized in that they are negative for CD106, and in that the expression level of a metallothionein family gene is increased, in comparison to the expression level thereof in the cells before the treatment by the physical stimulation or chemical stimulation. The characteristics of the above-described mesenchymal stem cells having relatively high proliferative ability will be described later in the present description.

[3] Characteristics of mesenchymal stem cells in the present invention

The mesenchymal stem cells of the present invention are characterized in that they are negative for CD106, and in that the expression level of a metallothionein family gene is increased, in comparison to the expression level thereof in the cells before the treatment by the physical stimulation or chemical stimulation. Accordingly, the mesenchymal stem cells (mesenchymal stem cells having relatively high proliferative ability) of the present invention are mesenchymal stem cells, which are negative for CD106, and in which the expression level of a metallothionein family gene is increased, in comparison to the expression level thereof in the cells before the treatment by the physical stimulation or chemical stimulation.

In the present invention, an expression marker for MSCs can be detected by any given detection method known in the present technical field. Examples of the expression marker include a cell surface antigen and an intracellularly expressed protein, but are not limited thereto. Specific examples of the expression marker include CD106 (VCAM1), CD105 (Endoglin), CD73 (Ecto-5'-nucleotidase), CD90 (Thy-1), CD45 (Leukocyte Common Antigen), CD34 (Hematopoietic Progenitor Cell Antigen), CD11b (Integrin αM), CD79alpha (Mb-1), HLA-DR (Human Leukocyte Antigen DR), CD324 (E-cadherin), and CD326 (EpCAM).

The mesenchymal stem cells of the present invention are negative for CD106. The mesenchymal stem cells of the present invention are preferably positive for CD105, CD73, and CD90, and negative for CD45, CD34, CD11b, CD79alpha, and HLA-DR.

Examples of the above-described method of detecting an expression marker include flow cytometry and cell staining, but are not limited thereto. When cells emitting stronger fluorescence than a negative control (isotype control) are detected in flow cytometry using a fluorescently labeled antibody, the cells are determined to be "positive" for the marker. Any given antibody known in the present technical field can be used as such a fluorescently labeled antibody, and examples of the fluorescently labeled antibody include antibodies labeled with fluorescein isothiocyanate (FITC), phycoerythrin (PE), allophycocyanin (APC), etc., but are not limited thereto. When stained cells or cells emitting fluorescence are observed under a microscope in cell staining, the cells are determined to be "positive" for the marker. The cell staining may be either immune cell staining using antibodies, or non-immune cell staining without using antibodies.

The timing of detecting the above-described expression marker is not particularly limited, and it is, for example, before the cells are treated by physical stimulation or chemical stimulation (e.g., immediately after separation of cells from a biological sample, immediately after completion of the pre-culture, immediately before the selection step, etc.), after the cells have been treated by physical stimulation or chemical stimulation (e.g., immediately after completion of the selection step, immediately after completion of the main culture, immediately after an N number of subcultures after completion of the main culture (wherein N indicates an integer of 1 or greater), etc.), or before formulating the cells as a pharmaceutical product composition.

The mesenchymal stem cells of the present invention are preferably negative for SA-β-Gal (senescence-associated beta-galactosidase). As described in the after-mentioned Examples, the present inventors found that MSCs having relatively low proliferative ability are senescent cells that are positive for SA-β-Gal, whereas MSCs having relatively high proliferative ability are non-senescent cells that are negative for SA-β-Gal. MSCs negative for SA-β-Gal mean that the cells are MSCs having relatively high proliferative ability.

The above-described SA-β-Gal can be detected, for example, by detecting the activity of SA-β-Gal, and specifically, such detection can be carried out by cell staining using a SA-β-Gal-specific substrate (e.g., a chromogenic substrate or a fluorescent substrate). In the cell staining with a SA-β-Gal-specific substrate, when intracytoplasmically stained cells are observed under an optical microscope, the cells are determined to be "positive" for SA-β-Gal.

The timing of detecting the above-described SA-β-Gal is not particularly limited, and it is, for example, before the cells are treated by physical stimulation or chemical stimulation (e.g., immediately after separation of cells from a biological sample, immediately after completion of the pre-culture, immediately before the selection step, etc.), after the cells have been treated by physical stimulation or chemical stimulation (e.g., immediately after completion of the selection step, immediately after completion of the main culture, immediately after an N number of subcultures after completion of the main culture (wherein N indicates an integer of 1 or greater), etc.), or before formulating the cells as a pharmaceutical product composition.

In the mesenchymal stem cells of the present invention, the expression level of a metallothionein family gene is increased, in comparison to the expression level thereof in the cells before the treatment by the physical stimulation or chemical stimulation. Metallothionein is a low-molecular-weight metal-binding protein (molecular weight: approximately 6,500) comprising many cysteine residues, and the presence thereof has been confirmed in many organism species. Four types of subtypes (MT-I, MT-II, MT-III, and MT-IV) are present in mammals. Known physiological functions of metallothionein-I and -II include a reduction in the toxicity of heavy metal, accumulation of heavy metal, metabolic regulation of zinc and copper, antioxidative action, scavenging action on free radicals, a reduction in the side effects of an anticancer agent, and anticancer agent resistance. In the present invention, since the expression level of a metallothionein family gene is increased by treating mesenchymal stem cells by physical stimulation or chemical stimulation, specifically, for example, by a freezing-thawing treatment, it is assumed that the improvement of the proliferative ability of mesenchymal stem cells and the improvement of the resistance of mesenchymal stem cells to freezing-thawing have been achieved. However, the present invention is not limited at all by the above-described theory or mechanism.

Preferably, examples of the metallothionein family gene include at least one, at least two, at least three, at least four, at least five, or all of six selected from the group consisting of MT1E, MT1F, MT1G, MT1H, MT1X, and MT2A.

MT1E is a gene consisting of the nucleotide sequence shown in SEQ ID NO: 1, or a gene encoding a polypeptide consisting of the amino acid sequence shown in SEQ ID NO: 7.

MT1F is a gene consisting of the nucleotide sequence shown in SEQ ID NO: 2, or a gene encoding a polypeptide consisting of the amino acid sequence shown in SEQ ID NO: 8.

MT1G is a gene consisting of the nucleotide sequence shown in SEQ ID NO: 3, or a gene encoding a polypeptide consisting of the amino acid sequence shown in SEQ ID NO: 9.

MT1H is a gene consisting of the nucleotide sequence shown in SEQ ID NO: 4, or a gene encoding a polypeptide consisting of the amino acid sequence shown in SEQ ID NO: 10.

MT1X is a gene consisting of the nucleotide sequence shown in SEQ ID NO: 5, or a gene encoding a polypeptide consisting of the amino acid sequence shown in SEQ ID NO: 11.

MT2A is a gene consisting of the nucleotide sequence shown in SEQ ID NO: 6, or a gene encoding a polypeptide consisting of the amino acid sequence shown in SEQ ID NO: 12.

In the mesenchymal stem cells of the present invention, the expression level of a metallothionein family gene is not particularly limited, as long as the expression level of the metallothionein family gene is increased in comparison to the expression level thereof in the cells before the treatment by the physical stimulation or chemical stimulation. Preferably, the expression level of the metallothionein family gene is increased at a level of 1.1 times or more, 1.2 times or more, 1.3 times or more, 1.4 times or more, 1.5 times or more, 1.6 times or more, 1.7 times or more, 1.8 times or more, 1.9 times or more, 2.0 times or more, 2.1 times or more, or 2.2 times or more. The phrase "the expression level of a metallothionein family gene" is used herein to mean the expression level measured by any one of the after-mentioned methods, or the expression level measured by other methods. The measurement method is not particularly limited.

The above-described expression level of a metallothionein family gene can be measured using, as an indicator, the transcriptional product (mRNA) of a metallothionein family gene, or a metallothionein family protein. Specifically, the expression level of a metallothionein family gene can be measured by RT-PCR, Northern blot hybridization, microarray analysis involving hybridization performed on a microarray, or immunoassay (e.g., ELISA (enzyme-linked immunosorbent assay)), etc.

The above-described microarray analysis involving hybridization performed on a microarray can be specifically carried out by the following procedures (1) to (4):

(1) Adhered cells are non-enzymatically peeled from a plastic culture vessel using Cell Scraper (manufactured by Corning), and are then recovered by centrifugation. Using a RNA stabilization reagent (RNAlater (manufactured by Thermo Fisher Scientific)), the cells are stably preserved, and then, using an RNA extraction kit (RNeasy Plus Mini Kit (manufactured by QIAGEN)), total RNA is extracted and purified.
(2) Using the purified total RNA as a template, cDNA is synthesized by a reverse transcription reaction, and further, cRNA is then transcribed from the synthesized cDNA by *in vitro* transcription, followed by labeling with biotin.
(3) The biotin-labeled cRNA is added to a hybridization buffer, and hybridization is then carried out on Human GeneGenome U133A 2.0 Array (manufactured by Affymetrix) for 16 hours. The resultant is washed with GeneChip Fluidics Station 450 (manufactured by Affymetrix), is then stained with phycoerythrin, and is then scanned using GeneChip Scanner 3000 7G (manufactured by Affymetrix). The image is analyzed using AGCC (Affymetrix GeneChip Command Console Software) (manufactured by Affymetrix), and is then numerized using Affymetrix Expression Console (manufactured by Affymetrix).
(4) Numerized data files from two arrays were compared and analyzed using the analysis software GeneSpring GX (manufactured by Agilent Technologies). The normalized value of a sample of the mesenchymal stem cells is divided by the normalized value of a sample of the cells before treatment by physical stimulation or chemical stimulation (control), to calculate a Fold Change value.

The resistance of the mesenchymal stem cells of the present invention to freezing-thawing is improved, in comparison to the cells before treatment by physical stimulation or chemical stimulation, which will be described in detail in the Examples. The resistance of the mesenchymal stem cells to freezing-thawing can be evaluated, for example, using the survival rate of the cells after cryopreservation and thawing as an indicator. Specifically, the resistance of the cells to freezing-thawing can be evaluated by leaving at rest the cells, which have been cryopreserved and then thawed, at a predetermined temperature (e.g., 25°C (room temperature) or 37°C (body temperature)) for a predetermined period of time (e.g., 0, 1 or 2 hours), then staining the cells with trypan blue, and then measuring the cell survival rate using a hemocytometer. However, the evaluation method is not limited thereto.

The present inventors found that there may be a case where the average diameter of MSCs having relatively high proliferative ability in a floating state is smaller than the average diameter of MSCs having relatively low proliferative ability in a floating state. The method of measuring the diameters of MSCs in a floating state is not particularly limited, and such diameters can be measured, for example, by observation under a phase-contrast microscope, or using an automatic cell counter. The average diameter of MSCs in a floating state is calculated as a mean value of the diameters of a plurality of cells measured by the above-described measurement method.

The diameters of MSCs having relatively high proliferative ability in a floating state in the present invention may be, for example, 80% or less, 75% or less, 70% or less, or 65% or less of the average diameter of the MSCs having relatively low proliferative ability in a floating state, but are not limited thereto. On the other hand, the average diameter of MSCs having relatively high proliferative ability in a floating state may be, for example, 45% or more, 50% or more, or 55% or more of the diameters of MSCs having relatively low proliferative ability in a floating state, but is not particularly limited thereto.

The average diameter of MSCs having relatively high proliferative ability in a floating state in the present invention may be, for example, 28 μm or less, 27 μm or less, 26 μm or less, 25 μm or less, 24 μm or less, 23 μm or less, 22 μm or less, 21 μm or less, 20 μm or less, 19 μm or less, 18 μm or less, 17 μm or less, 16 μm or less, 15 μm or less, or 14 μm or less, but is not particularly limited thereto. On the other hand, the average diameter of MSCs having relatively high proliferative ability in a floating state may be, for example, 8 μm or more, 9 μm or more, or 10 μm or more, but is not particularly limited thereto.

The present inventors found that there may be a case where the average length of the major axes of MSCs having relatively high proliferative ability in an adhesion state tends to be smaller than the average length of the major axes of MSCs having relatively low proliferative ability in an adhesion state, but the present invention is not particularly limited to this finding. In addition, the present inventors also found that there may be a case where the average length of the minor axes of MSCs having relatively high proliferative ability in an adhesion state tends to be smaller than the average length of the minor axes of MSCs having relatively low proliferative ability in an adhesion state, but the present invention is not particularly limited to this finding. The method of measuring the length of the major axis or minor axis of MSC in an adhesion state is not particularly limited, and it can be measured, for example, by measuring the length of the major axis or minor axis of the cells adhering to a plastic culture vessel by microscopic observation. The average length of the major axes or minor axes of MSCs in an adhesion state is calculated as a mean value of the major axes or minor axes of a plurality of cells measured by the above-described measurement method.

The average length of the major axes of MSCs having relatively high proliferative ability in an adhesion state in the present invention may be, for example, 80% or less, 70% or less, 60% or less, 50% or less, or 40% or less of the average length of the major axes of MSCs having relatively low proliferative ability in an adhesion state, but is not particularly limited thereto. On the other hand, the average length of the major axes of MSCs having relatively high proliferative ability in an adhesion state may be, for example, 20% or more, 25% or more, 30% or more, or 35% or more of the average length of the major axes of MSCs having relatively low proliferative ability in an adhesion state, but is not particularly limited thereto.

The average length of the major axes of MSCs having relatively high proliferative ability in an adhesion state in the present invention may be, for example, 13 μm or more, 14 μm or more, 15 μm or more, or 16 μm or more, but is not particularly limited thereto. On the other hand, the average length of the major axes of MSCs having relatively high proliferative ability in an adhesion state may be, for example, 48 μm or less, 46 μm or less, or 44 μm or less, but is not particularly limited thereto.

The average length of the minor axes of MSCs having relatively high proliferative ability in an adhesion state in the present invention may be, for example, 80% or less, 70% or less, 60% or less, 50% or less, or 40% or less of the average length of the minor axes of MSCs having relatively low proliferative ability in an adhesion state, but is not particularly limited thereto. On the other hand, the average length of the minor axes of MSCs having relatively high proliferative ability in an adhesion state may be, for example, 25% or more, 30% or more, or 35% or more of the average length of the minor axes of MSCs having relatively low proliferative ability in an adhesion state, but is not particularly limited thereto.

The average length of the minor axes of MSCs having relatively high proliferative ability in an adhesion state in the present invention may be, for example, 4 μm or more, 5 μm or more, or 6 μm or more, but is not particularly limited thereto. On the other hand, the average length of the minor axes of MSCs having relatively high proliferative ability in an adhesion state may be, for example, 14 μm or less, 13 μm or less, or 12 μm or less, but is not particularly limited thereto.

The present inventors found that the specific growth rate of MSCs having relatively high proliferative ability is higher than the specific growth rate of MSCs having relatively low proliferative ability. That is to say, MSCs having relatively high proliferative ability are MSCs having a relatively high specific growth rate, and MSCs having relatively low proliferative ability are MSCs having a relatively low specific growth rate. The proliferative ability of MSCs can be evaluated using a specific growth rate. The specific growth rate is defined to be an increase in cell count per unit time, and it is represented by the formula: $\mu = \ln (mt2/mt1) / (t2 - t1)$. Herein, t1 and t2 indicate the number of culture days, mt1 indicates a cell count on the $t1^{th}$ day, and mt2 indicates a cell count on the $t2^{th}$ day (wherein $t2 > t1$). Accordingly, the unit of the specific growth rate is (cells/cells/day) = (1/day).

The specific growth rate of MSCs having relatively high proliferative ability in the present invention is higher than the specific growth rate of MSCs having relatively low proliferative ability at a magnification of preferably 1.1 times or more, more preferably 1.2 times or more, even more preferably 1.3 times or more, further preferably 1.4 times or more, still further preferably 1.5 times or more, still further preferably 1.6 times or more, still further preferably 1.7 times or more, still further preferably 1.8 times or more, still further preferably 1.9 times or more, still further preferably 2.0 times or more, still further preferably 2.1 times or more, still further preferably 2.2 times or more, still further preferably 2.3 times or more, still further preferably 2.4 times or more, still further preferably 2.5 times or more, still further preferably 2.6 times or more, still further preferably 2.7 times or more, still further preferably 2.8 times or more, still further preferably 2.9 times or more, still further preferably 3.0 times or more, still further preferably 3.1 times or more, still further preferably 3.2 times or more, still further preferably 3.3 times or more, still further preferably 3.4 times or more, and particularly preferably 3.5 times or more. In addition, the specific growth rate of MSCs having relatively high proliferative ability is not particularly limited, and it is, for example, 5.0 times or less, 4.5 times or less, or 4.0 times or less the specific growth rate of MSCs having relatively low proliferative ability.

The specific growth rate of MSCs having relatively high proliferative ability in the present invention is 0.07 (1/day) or more, preferably 0.08 (1/day) or more, more preferably 0.09 (1/day) or more, even more preferably 0.10 (1/day) or more, further preferably 0.11 (1/day) or more, still further preferably 0.12 (1/day) or more, still further preferably 0.13 (1/day) or more, still further preferably 0.14 (1/day) or more, still further preferably 0.15 (1/day) or more, still further preferably 0.16 (1/day) or more, still further preferably 0.17 (1/day) or more, still further preferably 0.18 (1/day) or more, still further preferably 0.19 (1/day) or more, still further preferably 0.20 (1/day) or more, still further preferably 0.21 (1/day) or more, still further preferably 0.22 (1/day) or more, still further preferably 0.23 (1/day) or more, still further preferably 0.24 (1/day) or more, still further preferably 0.25 (1/day) or more, still further preferably 0.26 (1/day) or more, still further preferably 0.27 (1/day) or more, still further preferably 0.28 (1/day) or more, still further preferably 0.29 (1/day) or more, still further preferably 0.30 (1/day) or more, still further preferably 0.31 (1/day) or more, still further preferably 0.32 (1/day) or more, still further preferably 0.33 (1/day) or more, still further preferably 0.34 (1/day) or more, still further preferably 0.35 (1/day) or more, still further preferably 0.36 (1/day) or more, still further preferably 0.37 (1/day) or more, still further preferably 0.38 (1/day) or more, and particularly preferably 0.39 (1/day) or more. In addition, the specific growth rate of MSCs having relatively high proliferative ability is not particularly limited, and it is, for example, 0.60 (1/day) or less, or 0.50 (1/day) or less.

The possible subculture number of MSCs having relatively high proliferative ability in the present invention is 1 or more, preferably 2 or more, more preferably 3 or more, even more preferably 4 or more, further preferably 5 or more, still further preferably 6 or more, still further preferably 7 or more, still further preferably 8 or more, still further preferably 9 or more, still further preferably 10 or more, still further preferably 11 or more, still further preferably 12 or more, still further preferably 13 or more, still further preferably 14 or more, still further preferably 15 or more, still further preferably 16 or more, still further preferably 17 or more, still further preferably 18 or more, still further preferably 19 or more, still further preferably 20 or more, and still further preferably 25 or more. In addition, the upper limit of the possible subculture number is not particularly limited, and it is, for example, 50 or less, 45 or less, 40 or less, 35 or less, or 30 or less.

According to the production method of the present invention, MSCs having relatively high proliferative ability can be obtained, and thereby, large amounts of cell formulations (pharmaceutical compositions) can be promptly produced. The lower limit of the cell count obtained per batch culture (the cell count obtained per unit surface area per unit culture day) is different depending on a seeded cell count, a seeding density, etc. It is, for example, $2.3 \times 10^3$ (cells/$cm^2$/day) or more, $2.4 \times 10^3$ (cells/$cm^2$/day) or more, $2.5 \times 10^3$ (cells/$cm^2$/day) or more, $2.6 \times 10^3$ (cells/$cm^2$/day) or more, $2.7 \times 10^3$ (cells/$cm^2$/day) or more, $2.8 \times 10^3$ (cells/$cm^2$/day) or more, $2.9 \times 10^3$ (cells/$cm^2$/day) or more, $3.0 \times 10^3$ (cells/$cm^2$/day) or more, $3.1 \times 10^3$ (cells/$cm^2$/day) or more, or $3.2 \times 10^3$ (cells/$cm^2$/day) or more. On the other hand, the upper limit of the cell count obtained per batch culture is not particularly limited, and it is, for example, $4.0 \times 10^3$ (cells/$cm^2$/day) or less, $3.9 \times 10^3$ (cells/$cm^2$/day) or less, $3.8 \times 10^3$ (cells/$cm^2$/day) or less, $3.7 \times 10^3$ (cells/$cm^2$/day) or less, $3.6 \times 10^3$ (cells/$cm^2$/day) or less, $3.5 \times 10^3$ (cells/$cm^2$/day) or less, $3.4 \times 10^3$ (cells/$cm^2$/day) or less, or $3.3 \times 10^3$ (cells/$cm^2$/day) or less.

The mesenchymal stem cells of the present invention may be provided in the form of a composition comprising a combination of the mesenchymal stem cells with a medium. As such a medium, a liquid medium (e.g., a medium, the after-mentioned pharmaceutically acceptable medium, etc.) can be preferably used.

[4] Cell population comprising mesenchymal stem cells

According to the present invention, provided is a cell population comprising mesenchymal stem cells, wherein the expression level of a metallothionein family gene in the mesenchymal stem cells is increased, in comparison to the expression level thereof in the cells before the treatment by the physical stimulation or chemical stimulation, and wherein the mesenchymal stem cells are negative for CD 106.

In the above-described cell population, the rate of CD106-positive mesenchymal stem cells is preferably less than 5%, more preferably less than 4%, even more preferably less than 3%, further preferably less than 2%, and still further preferably less than 1%, and it may also be 0%.

In the above-described cell population, the rate of CD106-negative mesenchymal stem cells is preferably 95% or more, more preferably 96% or more, even more preferably 97% or more, further preferably 98% or more, and still further preferably 99% or more, and it may also be 100%.

The cell population of the present invention may comprise at least CD90-positive mesenchymal stem cells. The ratio of CD90-positive mesenchymal stem cells in the cell population is preferably 70% or more, more preferably 75% or more, even more preferably 80% or more, further preferably 90% or more, still further preferably 91% or more, still further preferably 92% or more, still further preferably 93% or more, still further preferably 94% or more, still further preferably 95% or more, still further preferably 96% or more, still further preferably 97% or more, still further preferably 98% or more, and still further preferably 99% or more, and it may also be 100%.

The cell population of the present invention may comprise at least CD73-positive mesenchymal stem cells. The ratio of CD73-positive mesenchymal stem cells in the cell population is preferably 55% or more, more preferably 60% or more, even more preferably 65% or more, further preferably 70% or more, still further preferably 75% or more, still further preferably 80% or more, still further preferably 85% or more, still further preferably 90% or more, still further preferably 95% or more, still further preferably 96% or more, still further preferably 97% or more, still further preferably 98% or more, and still further preferably 99% or more, and it may also be 100%.

The method of measuring the rate of cells that are positive for the above-described expression marker in the above-described cell population is, for example, flow cytometry, but is not limited thereto. In flow cytometry using a fluorescently labeled antibody, when cells emitting stronger fluorescence than a negative control (isotype control), the cells are determined to be "positive" for the marker. The rate of mesenchymal stem cells positive for a specific expression marker in the cell population can be calculated by the following procedures (1) to (3):

(1) The measurement results are developed on a histogram, in which the longitudinal axis indicates cell count, and the horizontal axis indicates the fluorescence intensity of a dye used to label an antibody.
(2) Fluorescence intensity, at which a cell population having stronger fluorescence intensity accounts for 0.1% to 1.0% in all cells measured with an isotype control antibody, is determined.
(3) The rate of cells having fluorescence intensity higher than the fluorescence intensity determined in the above (2) in all cells measured by an antibody reacting against a specific expression marker is calculated.

As a fluorescently labeled antibody, any given antibody known in the present technical field can be used, and examples of the fluorescently labeled antibody include antibodies labeled with fluorescein isothiocyanate (FITC), phycoerythrin (PE), and allophycocyanin (APC), but are not limited thereto.

Moreover, another method of measuring the rate of cells positive for the above-described expression marker in the above-described cell population is, for example, cell staining, but is not limited thereto. When stained cells or cells emitting fluorescence are observed under a microscope in the cell staining, the cells are determined to be "positive" for the marker. By observing a cell population under a microscope at the same magnification, and then obtaining the rate of the count of positive cells to a total cell count in the visual field, the rate of cells positive for a specific expression marker in the cell population can be determined. The cell staining may be either immune cell staining using antibodies, or non-immune cell staining without using antibodies.

MSCs having relatively high proliferative ability in the cell population of the present invention are preferably negative for SA-β-Gal (senescence-associated beta-galactosidase). As described in the after-mentioned Examples, the present inventors found that MSCs having relatively low proliferative ability are senescent cells positive for SA-β-Gal, whereas MSCs having relatively high proliferative ability are non-senescent cells negative for SA-β-Gal. The fact that MSCs are negative for SA-β-Gal means that the cells are MSCs having relatively high proliferative ability.

The average rate of SA-β-Gal-negative mesenchymal stem cells in the above-described cell population is preferably 90% or more, more preferably 91% or more, even more preferably 92% or more, further preferably 93% or more, still further preferably 94% or more, still further preferably 95% or more, still further preferably 96% or more, still further preferably 97% or more, still further preferably 98% or more, still further preferably 99% or more, and still further preferably 99.5% or more, and it may also be 100%.

With regard to the method of measuring the average rate of the cells positive for SA-β-Gal in the cell population, for example, the measurement can be carried out by detecting the activity of SA-β-Gal, and specifically, the measurement can be carried out by cell staining using a SA-β-Gal-specific substrate (e.g., a chromogenic substrate or a fluorescent substrate). In such cell staining using a SA-β-Gal-specific substrate, when intracytoplasmically stained cells are observed under an optical microscope, the cells are determined to be "positive" for SA-β-Gal. Moreover, the average rate of SA-β-Gal-negative cells in the cell population (the arithmetic mean value of the rate of SA-β-Gal-negative cells in the cell population) is calculated by the following procedures (1) to (4):

(1) A cell population adhering to a plastic culture vessel is prepared, and cell staining is performed on the cell population, using a SA-β-Gal-specific substrate.
(2) After completion of the cell staining, at least three sites in the same plastic culture vessel as described above were observed under an optical microscope at the same magnification, and the total cell count and a SA-β-Gal-positive cell count are then measured in the visual field.
(3) Regarding individual measurement sites, the rate of SA-β-Gal-negative cells in the cell population is calculated according to the following formula:

Rate of SA-β-Gal-negative cells in cell population = 100 - {(SA-β-Gal-positive cell count/total cell count) x 100 (%)}.

(4) The average rate of SA-β-Gal-negative cells in the cell population (the arithmetic mean value of the rate of SA-β-Gal-negative cells in the cell population) is calculated.

The mesenchymal stem cells in the cell population of the present invention are not particularly limited, as long as the expression level of a metallothionein family gene is increased, in comparison to the expression level thereof in the cells before the treatment by the physical stimulation or chemical stimulation. The expression level of the metallothionein family gene is preferably increased, in comparison to the expression level thereof in the cells before the treatment by the physical stimulation or chemical stimulation, at a magnification of 1.1 times or more, 1.2 times or more, 1.3 times or more, 1.4 times or more, 1.5 times or more, 1.6 times or more, 1.7 times or more, 1.8 times or more, 1.9 times or more, 2.0 times or more, 2.1 times or more, or 2.2 times or more.

The above-described expression level of a metallothionein family gene in the cell population can be measured using, as an indicator, the transcriptional product (mRNA) of a metallothionein family gene, or a metallothionein family protein. Specifically, the expression level of a metallothionein family gene can be measured by RT-PCR, Northern blot hybridization, microarray analysis involving hybridization performed on a microarray, or immunoassay (e.g., ELISA (enzyme-linked immuno-sorbent assay)), etc.

The above-described microarray analysis involving hybridization performed on a microarray can be specifically carried out by the following procedures (1) to (4):

(1) An adhered cell population is non-enzymatically peeled from a plastic culture vessel using Cell Scraper (manufactured by Corning), and is then recovered by centrifugation. Using a RNA stabilization reagent (RNAlater (manufactured by Thermo Fisher Scientific)), the cells are stably preserved, and then, using an RNA extraction kit (RNeasy Plus Mini Kit (manufactured by QIAGEN)), total RNA is extracted and purified.

(2) Using the purified total RNA as a template, cDNA is synthesized by a reverse transcription reaction, and further, cRNA is transcribed from the synthesized cDNA by *in vitro* transcription, followed by labeling with biotin.

(3) The biotin-labeled cRNA is added to a hybridization buffer, and hybridization is then carried out on Human GeneGenome U133A 2.0 Array (manufactured by Affymetrix) for 16 hours. The resultant is washed with GeneChip Fluidics Station 450 (manufactured by Affymetrix), is then stained with phycoerythrin, and is then scanned using GeneChip Scanner 3000 7G (manufactured by Affymetrix). The image is analyzed using AGCC (Affymetrix GeneChip Command Console Software) (manufactured by Affymetrix), and is then numerized using Affymetrix Expression Console (manufactured by Affymetrix).

(4) Numerized data files from two arrays were compared and analyzed using the analysis software GeneSpring GX (manufactured by Agilent Technologies). The normalized value of a sample of the mesenchymal stem cells is divided by the normalized value of a sample of the cells before treatment by physical stimulation or chemical stimulation (control), to calculate a Fold Change value.

Preferably, the resistance of the mesenchymal stem cells in the cell population of the present invention to freezing-thawing is improved, in comparison to the cells before the treatment by the physical stimulation or chemical stimulation. The resistance of the mesenchymal stem cells to freezing-thawing can be evaluated, for example, using the survival rate of the cells after cryopreservation and thawing as an indicator. Specifically, the resistance of the cells to freezing-thawing can be evaluated by leaving at rest the cells, which have been cryopreserved and then thawed, at a predetermined temperature (e.g., 25°C (room temperature) or 37°C (body temperature)) for a predetermined period of time (e.g., 0, 1 or 2 hours), then staining the cells with trypan blue, and then measuring the cell survival rate using a hemocytometer. However, the evaluation method is not limited thereto.

The present inventors found that there may be a case where the average diameter of MSCs having relatively high proliferative ability in a floating state is smaller than the average diameter of MSCs having relatively low proliferative ability in a floating state. The method of measuring the diameters of MSCs in a floating state is not particularly limited, and such diameters can be measured, for example, by observation under a phase-contrast microscope, or using an automatic cell counter. The average diameter of MSCs in a floating state is calculated as a mean value of the diameters of a plurality of cells measured by the above-described measurement method.

The average diameter of MSCs having relatively high proliferative ability in a floating state in the mesenchymal stem cell population of the present invention may be, for example, 28 μm or less, 27 μm or less, 26 μm or less, 25 μm or less, 24 μm or less, 23 μm or less, 22 μm or less, 21 μm or less, 20 μm or less, 19 μm or less, 18 μm or less, 17 μm or less, 16 μm or less, 15 μm or less, or 14 μm or less, but is not particularly limited thereto. On the other hand, the average diameter of MSCs having relatively high proliferative ability in a floating state may be, for example, 8 μm or more, 9 μm or more, or 10 μm or more, but is not particularly limited thereto.

The present inventors found that there may be a case where the average length of the major axes of MSCs having relatively high proliferative ability in an adhesion state tends to be smaller than the average length of the major axes of MSCs having relatively low proliferative ability in an adhesion state. In addition, the present inventors also found that there may be a case where the average length of the minor axes of MSCs having relatively high proliferative ability in an adhesion state tends to be smaller than the average length of the minor axes of MSCs having relatively low proliferative ability in an adhesion state. The method of measuring the length of the major axis or minor axis of MSCs in an adhesion state is not particularly limited, and it can be measured, for example, by measuring the length of the major axis or minor axis of the cells adhering to a plastic culture vessel by microscopic observation. The average length of the major axes or minor axes of MSCs in an adhesion state is calculated as a mean value of the major axes or minor axes of a plurality of cells measured by the above-described measurement method.

The average length of the major axes of MSCs having relatively high proliferative ability in an adhesion state in the present invention may be, for example, 13 μm or more, 14 μm or more, 15 μm or more, or 16 μm or more, but is not particularly limited thereto. On the other hand, the average length of the major axes of MSCs having relatively high proliferative ability in an adhesion state may be, for example, 48 μm or less, 46 μm or less, or 44 μm or less, but is not particularly limited thereto.

The average length of the minor axes of MSCs having relatively high proliferative ability in an adhesion state in the cell population of the present invention may be, for example, 80% or less, 70% or less, 60% or less, 50% or less, or 40% or less of the average length of the minor axes of MSCs having relatively low proliferative ability in an adhesion state, but is not particularly limited thereto. On the other hand, the average length of the minor axes of MSCs having relatively high proliferative ability in an adhesion state may be, for example, 25% or more, 30% or more, or 35% or more of the average length of the minor axes of MSCs having relatively low proliferative ability in an adhesion state, but is not particularly limited thereto.

The average length of the minor axes of MSCs having relatively high proliferative ability in an adhesion state in the mesenchymal stem cell population may be, for example, 4 μm or more, 5 μm or more, or 6 μm or more, but is not particularly limited thereto. On the other hand, the average length of the minor axes of MSCs having relatively high proliferative ability in an adhesion state may be, for example, 14 μm or less, 13 μm or less, or 12 μm or less, but is not particularly limited thereto.

The present inventors found that the specific growth rate of MSCs having relatively high proliferative ability in the cell population of the present invention is higher than the specific growth rate of MSCs having relatively low proliferative ability therein. That is to say, the MSCs having relatively high proliferative ability in the cell population are MSCs having a relatively high specific growth rate, and the MSCs having relatively low proliferative ability in the cell population are MSCs having a relatively low specific growth rate. The proliferative ability of the MSCs in the cell population can be evaluated using a specific growth rate. The specific growth rate is defined to be an increase in cell count per unit time, and it is represented by the formula: $\mu = \ln (mt2/mt1) / (t2 - t1)$. Herein, t1 and t2 indicate the number of culture days, mt1 indicates a cell count on the t1$^{th}$ day, and mt2 indicates a cell count on the t2$^{th}$ day (wherein t2 > t1). Accordingly, the unit of the specific growth rate is (cells/cells/day) = (1/day).

The specific growth rate of MSCs having relatively high proliferative ability in the cell population of present invention is higher than the specific growth rate of MSCs having relatively low proliferative ability at a magnification of preferably 1.1 times or more, more preferably 1.2 times or more, even more preferably 1.3 times or more, further preferably 1.4 times or more, still further preferably 1.5 times or more, still further preferably 1.6 times or more, still further preferably 1.7 times or more, still further preferably 1.8 times or more, still further preferably 1.9 times or more, still further preferably 2.0 times or more, still further preferably 2.1 times or more, still further preferably 2.2 times or more, still further preferably 2.3 times or more, still further preferably 2.4 times or more, still further preferably 2.5 times or more, still further preferably 2.6 times or more, still further preferably 2.7 times or more, still further preferably 2.8 times or more, still further preferably 2.9 times or more, still further preferably 3.0 times or more, still further preferably 3.1 times or more, still further preferably 3.2 times or more, still further preferably 3.3 times or more, still further preferably 3.4 times or more, and particularly preferably 3.5 times or more. In addition, the specific growth rate of MSCs having relatively high proliferative ability is not particularly limited, and it is, for example, 5.0 times or less, 4.5 times or less, or 4.0 times or less the specific growth rate of MSCs having relatively low proliferative ability.

The specific growth rate of MSCs having relatively high proliferative ability in the cell population of the present invention is 0.07 (1/day) or more, preferably 0.08 (1/day) or more, more preferably 0.09 (1/day) or more, even more preferably 0.10 (1/day) or more, further preferably 0.11 (1/day) or more, still further preferably 0.12 (1/day) or more, still further preferably 0.13 (1/day) or more, still further preferably 0.14 (1/day) or more, still further preferably 0.15 (1/day) or more, still further preferably 0.16 (1/day) or more, still further preferably 0.17 (1/day) or more, still further preferably 0.18 (1/day) or more, still further preferably 0.19 (1/day) or more, still further preferably 0.20 (1/day) or more, still further preferably 0.21 (1/day) or more, still further preferably 0.22 (1/day) or more, still further preferably 0.23 (1/day) or more, still further preferably 0.24 (1/day) or more, still further preferably 0.25 (1/day) or more, still further preferably 0.26 (1/day) or more, still further preferably 0.27 (1/day) or more, still further preferably 0.28 (1/day) or more, still further preferably 0.29 (1/day) or more, still further preferably 0.30 (1/day) or more, still further preferably 0.31 (1/day) or more, still further preferably 0.32 (1/day) or more, still further preferably 0.33 (1/day) or more, still further preferably 0.34 (1/day) or more, still further preferably 0.35 (1/day) or more, still further preferably 0.36 (1/day) or more, still further preferably 0.37 (1/day) or more, still further preferably 0.38 (1/day) or more, and particularly preferably 0.39 (1/day) or more. In addition, the specific growth rate of MSCs having relatively high proliferative ability is not particularly limited, and it is, for example, 0.60 (1/day) or less, or 0.50 (1/day) or less.

The possible subculture number of MSCs having relatively high proliferative ability in the cell population of the present invention is 1 or more, preferably 2 or more, more preferably 3 or more, even more preferably 4 or more, further preferably 5 or more, still further preferably 6 or more, still further preferably 7 or more, still further preferably 8 or more, still further preferably 9 or more, still further preferably 10 or more, still further preferably 11 or more, still further preferably 12 or more, still further preferably 13 or more, still further preferably 14 or more, still further preferably 15 or more, still further preferably 16 or more, still further preferably 17 or more, still further preferably 18 or more, still further preferably 19 or more, still further preferably 20 or more, and still further preferably 25 or more. In addition, the upper limit of the possible subculture number is not particularly limited, and it is, for example, 50 or less, 45 or less, 40 or less, 35 or less, or 30 or less.

The content of MSCs having relatively high proliferative ability in the cell population of the present invention is preferably 10% or more, more preferably 20% or more, even more preferably 30% or more, further preferably 40% or more, still further preferably 50% or more, still further preferably 60% or more, still further preferably 70% or more, still further preferably 80% or more, still further preferably 90% or more, still further preferably 91% or more, still further preferably 92% or more, still further preferably 93% or more, still further preferably 94% or more, still further preferably 95% or more, still further preferably 96% or more, still further preferably 97% or more, still further preferably 98% or more, still further preferably 99% or more, and particularly preferably 100%.

The cell population of the present invention can be preserved in a frozen state until immediately before the use thereof. The cell population of the present invention may comprise any given components, as well as MSCs having relatively high proliferative ability. Examples of such components include salts, polysaccharides (e.g., HES, dextran, etc.), proteins (e.g., albumin, etc.), DMSO, and medium components (e.g., components comprised in an RPMI1640 medium, etc.), but are not limited thereto.

The cell population of the present invention may be provided in the form of a composition comprising a combination of the cell population with a medium. As such a medium, a liquid medium (e.g., a medium, the after-mentioned pharmaceutically acceptable medium, etc.) can be preferably used.

[5] Pharmaceutical composition

The mesenchymal stem cells having relatively high proliferative ability according to the present invention, or a cell population comprising the above-described mesenchymal stem cells, can be used as a pharmaceutical composition. The pharmaceutical composition of the present invention comprises the mesenchymal stem cells of the present invention, or the cell population of the present invention, and a pharmaceutically acceptable medium.

The pharmaceutical composition of the present invention can be used as a cell therapy agent, such as, for example, a therapeutic agent for intractable diseases.

The pharmaceutical composition of the present invention can be used as a therapeutic agent for a disease selected from immune-related disease, ischemic disease (lower limb ischemia, ischemic heart disease (myocardial infarction, etc.), coronary heart disease, cerebrovascular ischemia, renal ischemia, pulmonary ischemia, etc.), neurological diseases, graft versus host disease (GVHD), Crohn's disease, inflammatory bowel diseases including ulcerative colitis, connective tissue diseases including systemic lupus erythematosus, cerebral infarction, intracerebral hematoma, cerebral vasospasm, radiation enteritis, liver cirrhosis, stroke, atopic dermatitis, multiple sclerosis, rheumatoid arthritis, psoriasis, lupus erythematosus, diabetes, mycosis fungoides (Alibert-Bazin syndrome), scleroderma, diseases caused by degeneration and/or inflammation of connective tissues such as cartilage, eye disease, angiogenesis-associated diseases, congestive heart failure, cardiomyopathy, wound, epithelial damage, fibrosis, pulmonary disease, and cancer. Inflammation can be suppressed by administration of the pharmaceutical composition of the present invention at an applied dose, with which the effects of the pharmaceutical composition can be measured in a therapeutic site.

Moreover, according to the present invention, use of the mesenchymal stem cells or cell population of the present invention for the production of a pharmaceutical composition, is provided.

Furthermore, according to the present invention, the mesenchymal stem cells of the present invention or the cell population of the present invention used for a pharmaceutical composition, is provided. Further, according to the present invention, the mesenchymal stem cells of the present invention or the cell population of the present invention for use in the treatment of a disease selected from immune-related disease, ischemic disease (lower limb ischemia, ischemic heart disease (myocardial infarction), coronary heart disease, cerebrovascular ischemia, renal ischemia, pulmonary ischemia, etc.), neurological diseases, graft versus host disease (GVHD), Crohn's disease, inflammatory bowel diseases including ulcerative colitis, connective tissue diseases including systemic lupus erythematosus, cerebral infarction, intracerebral hematoma, cerebral vasospasm, radiation enteritis, liver cirrhosis, stroke, atopic dermatitis, multiple sclerosis, rheumatoid arthritis, psoriasis, lupus erythematosus, diabetes, mycosis fungoides (Alibert-Bazin syndrome), scleroderma, diseases caused by degeneration and/or inflammation of connective tissues such as cartilage, eye disease, angiogenesis-associated diseases, congestive heart failure, cardiomyopathy, wound, epithelial damage, fibrosis, pulmonary disease, and cancer, is provided. Still further, according to the present invention, the mesenchymal stem cells of the present invention or the cell population of the present invention, which are administered to a patient or a subject and are used for myocardial regeneration, production of cardiomyocytes, angiogenesis, vascular repair, or suppression of the immune response, is provided.

Still further, according to the present invention, a method for transplanting the mesenchymal stem cells of the present invention or the cell population of the present invention to a patient or a subject, and a method for treating a disease of a patient or a subject, both of which comprise a step of administering a therapeutically effective amount of the mesenchymal stem cells of the present invention or the cell population of the present invention to the patient or the subject, is provided.

The dose of the pharmaceutical composition of the present invention is determined based on the amount of cells that allows a patient or a subject, who has been administered with the pharmaceutical composition, to obtain therapeutic effects, compared with a patient or a subject who has not been administered with the pharmaceutical composition. A specific dose can be appropriately determined depending on the form of administration, route of administration, intended use, and patient's or subject's age, body weight, and symptoms, and the like. The dose is not particularly limited, and it is, for example, $10^4$ cells/kg body weight or more, $10^5$ cells/kg body weight or more, or $10^6$ cells/kg body weight or more. On the other hand, the dose is not particularly limited, and it is, for example, $10^9$ cells/kg body weight or less, $10^8$ cells/kg body weight or less, or $10^7$ cells/kg body weight or less.

The method for administering the pharmaceutical composition of the present invention is not particularly limited. Examples of the form of administration include subcutaneous injection, intra-lymph nodal injection, intravenous injection, intravenous drip infusion, intraperitoneal injection, intrathoracic injection, direct localized injection, and direct localized transplantation. Known methods for administering the pharmaceutical composition include intravenous injection, intravenous drip infusion, direct localized injection, and direct localized transplantation, which are described, for example, in JP 2015-61520 A, Onken JE, et al. American College of Gastroenterology Conference 2006, Las Vegas, NV, Abstract 121., Garcia-Olmo D, et al. Dis Colon Rectum 2005; 48: 1416-23. The pharmaceutical composition of the present invention can also be administered by various types of methods described in the aforementioned publications.

The pharmaceutical composition of the present invention can also be used as an injection preparation, or a transplant preparation having a cell aggregate or sheet-like structure, for the treatment of other diseases, or a gel preparation mixed with any gel.

The patient or subject of the present invention is typically a human, but it may also be another animal. Examples of such another animal include mammals such as a dog, a cat, a bovine, a horse, a swine, a goat, sheep, a monkey (a crab-eating monkey, a Rhesus monkey, a common marmoset, or a Japanese macaque), a ferret, a rabbit, and rodents (a mouse, a rat, a gerbil, a Guinea pig, or a hamster), and birds such as a chicken and a quail, but are not particularly limited thereto.

The pharmaceutical composition of the present invention can be preserved in a frozen state until immediately before the use thereof. The pharmaceutical composition of the present invention may comprise any given components, which are used upon the treatment of a human. Examples of such components include salts, polysaccharides (e.g., HES, dextran, etc.), proteins (e.g., albumin, etc.), DMSO, and medium components (e.g., components comprised in an RPMI1640 medium, etc.), but are not limited thereto.

Moreover, with regard to the pharmaceutical composition of the present invention, mesenchymal stem cells or a cell population comprised therein may be diluted with an infusion preparation used as a pharmaceutically acceptable medium. The "infusion preparation (pharmaceutically acceptable medium)" in the present description is not particularly limited, as long as it is a solution used upon the treatment of a human, and examples of the infusion preparation include normal saline, 5% glucose solution, Ringer’s solution, lactated Ringer’s solution, acetated Ringer’s solution, initiation solution (No. 1 solution), rehydration solution (No. 2 solution), maintenance transfusion (No. 3 solution), and postoperative recovery solution (No. 4 solution).

Besides, with regard to the medical use of stem cells such as mesenchymal stem cells, the treatment of immune-related diseases and disorders, angiogenesis, production, repair and/or maintenance of connective tissue, the treatment of eye disease and excess angiogenesis, myocardial regeneration, joint repair, the treatment of genetic diseases and disorders, and immunomodulation have been known, and described, for example, in JP 2012-509087 A, JP 2014-501249 A, JP 2013-256515 A, JP 2014-185173 A, JP 2015-038059 A, JP 2015-110659 A, JP 2006-521121 A, JP 2009-542727 A, JP 2010-535715 A, JP 2014-224117 A, JP 2015-061862 A, JP 2002-511094 A, JP 2004-507454 A, JP 2010-505764 A, JP 2011-514901 A, JP 2013-064003 A, JP 2015-131795 A, and the like. Moreover, JP 2010-518096 A discloses the treatment of inflammatory diseases using placental stem cells, and JP 2015-178498 A and Japanese Patent No. 5950577 A disclose the treatment of stroke using placental stem cells. Furthermore, J Am Coll Cardiol. 2009 December 8; 54(24): 2277-2286. discloses the treatment of heart failure using bone marrow mesenchymal stem cells; Brain 2011: 134; 1790-1807. discloses the treatment of cerebral infarction using bone marrow mesenchymal stem cells; and Ann Thorac Surg, 2013; 95: 1827-33. discloses the treatment of heart failure using placental mesenchymal stem cells. The mesenchymal stem cells or cell population of the present invention are also useful for the treatment of various types of diseases described in the above-described publications.

The present invention is specifically explained with reference to the Examples below; however, the present invention is not limited to the Examples.

## Examples

### (Example 1)

Amniotic MSCs having a high specific growth rate (amniotic MSCs having relatively high proliferative ability) were obtained by a series of Step 1-1 to Step 1-6, as described below.

(Step 1-1: Collection of amnion)

An fetal membrane and a placenta were aseptically collected as fetal appendages from a pregnant woman with an elective Caesarean section case, after the obtaining of informed consent. The obtained fetal membrane and placenta were placed in a sterilized tray filled with a normal saline, and amnion was then manually peeled from the edge of the fetal membrane. The amnion was washed twice with a Hanks’ balanced salt solution (free of Ca and Mg), and adhered blood and clots were then removed from the amnion.

(Step 1-2: Enzyme treatment of amnion and recovery of amniotic MSCs)

(Method)

5 mL of a Hanks' balanced salt solution (containing Ca and Mg) comprising 300 CDU/mL purified collagenase (Worthington, CLSPA) and 200 PU/mL dispase I (Wako Pure Chemical Industries, Ltd., Product No. 386-02271) was added per 1 g of amnion, and the obtained mixture was then shaken and stirred under conditions of 37°C, 90 minutes and 60 rpm. Herein, CDU (collagen digestion unit) is defined as an enzyme amount necessary for generating amino acids and peptides corresponding to 1 μmol leucine at 37°C at pH 7.5 for 5 hours, using collagen as a substrate. In addition, PU (protease unit) is defined as an enzyme amount necessary for generating amino acids and peptides corresponding to 1 μg of tyrosine at 35°C at pH 7.2 for 1 minute, using lactic acid casein as a substrate. An enzymatically treated product of the amnion was suspended in two times its volume of αMEM (Alpha Modification of Minimum Essential Medium Eagle) supplemented with 10% fetal bovine serum (FBS). The obtained cell suspension was filtered through a nylon mesh filter (pore size: 100 μm), and a filtrate containing amniotic MSCs was then recovered. Undigested amnion remaining on the nylon mesh filter was evaluated by hematoxylin-eosin (HE) staining. The filtrate containing amniotic MSCs was centrifuged at 400 x g for 5 minutes, and a supernatant was then discarded. The obtained cell pellets were suspended in αMEM supplemented with 10% FBS, to obtain a cell suspension comprising amniotic MSCs having different proliferative ability. A Turk's staining solution was mixed with a portion of the obtained cell suspension, and a cell count was then measured. The amniotic MSCs recovered from the filtrate were stained with a labeled antibody at 4°C for 15 minutes, and propidium iodide or 7-AAD (7-Amino-Actinomycin D) was then added to the amniotic MSCs to remove dead cells. Thereafter, a surface antigen was analyzed using a flow cytometer (FACS Canto: Becton, Dickinson and Company, BD Japan (hereinafter abbreviated as "BD"). An anti-CD90-FITC antibody (BD) was used herein as a labeled antibody.

(Results)

As a result of the hematoxylin-eosin (HE) staining, it was found that an epithelial cell layer was undigested and maintained in the amnion remaining on the filter, and that large quantities of epithelial cells were present in the epithelial cell layer. Moreover, an extracellular matrix layer was digested in the amnion remaining on the filter, and there were found no amniotic MSCs on the filter. These results demonstrate that a filtrate containing almost no epithelial cells was recovered by adopting collagenase and dispase, which target the extracellular matrix layer of the amnion and do not target the epithelial cell layer thereof.

As a result of the measurement of a cell count by Turk's staining, it was confirmed that the cell count obtained from amniotic tissues was $2.3 \times 10^6$ cells per g of tissues.

As a result of the flow cytometry, a cell population comprising the cells recovered from the filtrate was positive for CD90. These results demonstrate that the cells recovered from the filtrate were mesenchymal stem cells.

(Step 1-3: Pre-culture 1)

A portion of the cell population obtained from the enzymatically treated product of the amnion was seeded on a plastic culture vessel, and was then cultured in αMEM supplemented with 10% FBS, until the confluent percentage became 90% or more and 95% or less. The cells obtained by this step are referred to as cells of pre-culture 1.

(Step 1-4: Pre-culture 2)

(Method)

The cells of pre-culture 1 were treated with ethylenediaminetetraacetic acid (EDTA), and were then treated with trypsin, so that the cells were peeled from the plastic culture vessel. The obtained cell suspension was centrifuged to remove a supernatant therefrom. The obtained cell pellets were suspended in αMEM supplemented with 10% FBS. The cells were seeded at a seeding density of $2.8 \times 10^3$ cells/cm$^2$ on a plastic culture vessel, and were then cultured in αMEM supplemented with 10% FBS for 8 days. The cells obtained by this pre-culture are referred to as cells of pre-culture 2. The cells of pre-culture 2 were used in the subsequent tests.

(Results)

After completion of the culture for 8 days, the cells of pre-culture 2 were obtained at a cell density of 4.6 x $10^3$ cells/$cm^2$. In this case, the specific growth rate of the cells of pre-culture 2 was 0.06 (1/day).

(Step 1-5: Stimulation treatment of amniotic MSCs by freezing-thawing)

(Method)

The cells of pre-culture 2 were treated with EDTA and were then treated with trypsin, so that the cells were peeled from the plastic culture vessel. The obtained cell suspension was centrifuged to remove a supernatant therefrom. The obtained cell pellets were suspended in an RPMI1640 medium supplemented with 0.5% (w/v) human serum albumin (HSA) (manufactured by Benesis Corporation, Albumin 25% Injection 12.5 g/50 ml), to a concentration of 2.0 x $10^7$ cells/mL or less. A cryopreservation solution comprising 12% (w/v) hydroxyethyl starch (HES) (manufactured by Nipro Corporation; Product No. HES40), 10% (w/v) dimethyl sulfoxide (DMSO) (manufactured by Sigma-Aldrich, Product No. D2650), and 8% (w/v) human serum albumin (HSA) (manufactured by Benesis Corporation, Albumin 25% Injection 12.5 g/50 ml) was prepared. The cell suspension was mixed with an equal amount of the cryopreservation solution on ice, and 1 mL of the obtained mixture was transferred into a cryotube. Using a program freezer, the cryotube was cooled to -40°C at a freezing rate of -2°C/min or more and -1°C/min or less, and was then cooled to -90°C at a freezing rate of -10°C/min. Thereafter, the cryotube was cryopreserved at -150°C in an ultra-low temperature freezer for 2 days. Thereafter, the thus cryopreserved cryotube was immersed in a thermostatic bath at 37°C, so that it was promptly melted. A portion of the melted cell suspension was collected, and was then stained with trypan blue. After that, a cell count, a survival rate, and a size were measured using a hemocytometer.

(Results)

As shown in Fig. 1 and Table 1, immediately after completion of the freezing-thawing treatment, amniotic MSCs in a floating state included two types of cells (i.e., cells having a relatively large size and cells having a relatively small size), namely, a cell group having a diameter of 19.9 ± 3.1 μm (i.e., average diameter: 19.9 μm) and a cell group having a diameter of 12.0 ± 1.9 μm (i.e., average diameter: 12.0 μm). The cell survival rate immediately after completion of the freezing-thawing treatment was 55%, and the content of cells having a relatively small size (in viable cells), namely, the rate of the number of cells having a relatively small size / the number of cells having a relatively large size was 2% (i.e., the number of cells having a relatively small size / the number of cells having a relatively large size = 2/98).

Moreover, the cell survival rate in the cells subjected to the freezing-thawing treatment (the cells of pre-culture 2) was 91%, and the content of cells having a relatively small size (in viable cells) was 1% (i.e., the number of cells having a relatively small size / the number of cells having a relatively large size = 1/99).

From the above-described results, it was found that the survival rate of amniotic MSCs was decreased as a result of the freezing-thawing treatment, and that such a decrease in the survival rate was caused by the death of the cells having a relatively large size (the cells having a large diameter).

[Table 1]

Table 1. Diameter of amniotic MSCs in a floating state

| | Diameter in floating state (μm) |
|---|---|
| Cells having a relatively large size | 19.9 ± 3.1 |
| Cells having a relatively small size | 12.0 ± 1.9 |

(Step 1-6: Main culture)

(Method)

Amniotic MSCs after completion of the freezing-thawing treatment were cultured as follows. The melted cell suspension was centrifuged to remove a supernatant therefrom. The obtained cell pellets were suspended in αMEM supplemented with 10% FBS. A portion of the obtained cell suspension was seeded on a plastic culture vessel, and was then cultured in αMEM supplemented with 10% FBS, until the confluent percentage became 90% or more and 95% or less. The cells obtained by this culture are referred to as cells of main culture 1.

Adhered cells of main culture 1 were treated with EDTA and were then treated with trypsin, so that the cells were peeled from the plastic culture vessel. The obtained cell suspension was centrifuged to remove a supernatant therefrom. The obtained cell pellets were suspended in αMEM supplemented with 10% FBS. A portion of the obtained cell suspension was seeded on a 6300-$cm^2$ (total culture surface area) plastic culture vessel, and was then cultured in αMEM supplemented with 10% FBS, until the confluent percentage became 90% or more and 95% or less. The cells obtained as a result of the subculture and culture are referred to as cells of subculture 1. By an EDTA-trypsin treatment and centrifugation, cells of subculture 1 were recovered.

A portion was collected from each of the cells of main culture 1 and the cells of subculture 1, and was then stained with trypan blue. Thereafter, a cell count and a survival rate were measured using a hemocytometer. Using Cellular Senescence Detection Kit (SA-β-Gal Staining) (Cell Biolabs), the cells of main culture 1 and the cells of subculture 1 were subjected to a cellular senescence assay. In addition, a portion was collected from each of the cells of main culture 1 and the cells of subculture 1, it was then stained with a labeled antibody at 4°C for 15 minutes, and propidium iodide or 7-AAD (7-Amino-Actinomycin D) was then added to the cells to remove dead cells. Thereafter, a surface antigen was analyzed using a flow cytometer (FACS Canto: BD). An anti-CD90-FITC antibody (BD), an anti-CD105-FITC antibody (Ancell), an anti-CD73-PE antibody (BD), an anti-CD106-PE antibody (Miltenyi Biotec), an anti-CD324-PE antibody (BD), an anti-CD326-FITC antibody (BD), an anti-CD45-FITC antibody (BD), an anti-CD34-PE antibody (BD) and an anti-HLA-DR-FITC antibody (BD) were used herein as labeled antibodies described above. The rate of CD106-positive mesenchymal stem cells in the cell population was calculated by the following procedures (1) to (3):

(1) The measurement results were developed on a histogram, in which the longitudinal axis indicated cell count, and the horizontal axis indicated the fluorescence intensity of a pigment used to label an antibody.
(2) Fluorescence intensity, at which a cell population having stronger fluorescence intensity accounted for 0.1% to 1.0% in all cells measured with an isotype control antibody, was determined.
(3) The rate of cells having fluorescence intensity higher than the fluorescence intensity determined in the above (2) in all cells measured by an antibody against CD106 was calculated.

(Results)

The number of days, which was required for the cells after completion of the freezing-thawing treatment (the cells of main culture 1 and the cells of subculture 1) to reach a confluent percentage of 90% or more and 95% or less, was 10 days and 11 days, respectively. In these cases, the specific growth rate of the cells of main culture 1 was 0.20 (1/day), and the specific growth rate of the cells of subculture 1 was 0.19 (1/day) (Table 2). From these results, it was found that the specific growth rate of the amniotic MSCs that had been subjected to a freezing-thawing treatment was extremely high. The aforementioned results demonstrate that amniotic MSCs having a relatively small size are amniotic MSCs having a high specific growth rate (i.e., amniotic MSCs having relatively high proliferative ability). In addition, the cell count of the obtained cells of subculture 1 was $2.0 \times 10^8$ cells. From these results, it is found that $2.9 \times 10^3$ (cells/$cm^2$/day) cells (the cells of subculture 1) were obtained per batch culture. The aforementioned results demonstrate that amniotic MSCs necessary for the production of cell formulations can be promptly prepared and/or produced in large amounts.

[Table 2]

Table 2. Specific growth rate of amniotic MSCs

| | Cells of pre-culture 2 | Cells of main culture 1 | Cells of subculture 1 |
|---|---|---|---|
| Specific growth rate (1/day) | 0.06 | 0.20 | 0.19 |

Fig. 2, Fig. 3 and Fig. 4 show the phase-contrast microscopic images of adhered amniotic MSCs. Regardless of the presence or absence of a freezing-thawing treatment, the shape of amniotic MSCs in an adhesion state was a fibroblast-like spindle shape or small polygon.

Almost all amniotic MSCs, which had not been subjected to a freezing-thawing treatment (the cells of pre-culture 2), were cells having a major axis length of 78.4 ± 25.3 μm (i.e., average length of the major axes: 78.4 μm) and a minor axis length of 22.8 ± 6.0 μm (i.e., average length of the minor axes: 22.8 μm) (i.e., cells having a large major axis and a large minor axis, namely, cells having a relatively large size) in a confluent state (Table 3). As described above, the content of the cells having a relatively small size in the cells of pre-culture 2 was 1%.

In contrast, cells, which had been cultured until they became confluent after completion of the freezing-thawing treatment (cells of main culture 1), comprised not only the above-described cells having a relatively large size, but also cells having a major axis length of 30.1 ± 13.6 μm (i.e., average length of the major axes: 30.1 μm) and a minor axis length of 8.7 ± 2.5 μm (i.e., average length of the minor axes: 8.7 μm) (i.e., cells having a small major axis and a small minor axis, namely, cells having a relatively small size) (Table 3). The content of the cells having a relatively small size in the cells of main culture 1 was 90% (i.e., the number of the cells having a relatively small size / the number of the cells having a relatively large size = 90/10), and the content of the cells having a relatively small size in the cells of subculture 1 was 98% (i.e., the number of the cells having a relatively small size / the number of the cells having a relatively large size = 98/2).

From the aforementioned results, it is found that since the proliferative ability (specific growth rate) of the cells having a relatively large size was significantly reduced as a result of the freezing-thawing treatment, the cells having a relatively large size did not proliferate in culture, and the cells having a relatively small size mainly proliferated.

[Table 3]

Table 3. Lengths of major axis and minor axis of amniotic MSCs in adhesion state

| | Length of major axis (μm) | Length of minor axis (μm) |
|---|---|---|
| Cells having a relatively large size | 78.4 ± 25.3 | 22.8 ± 6.0 |
| Cells having a relatively small size | 30.1 ± 13.6 | 8.7 ± 2.5 |

In order to examine the possible subculture number, the cells of subculture 1 were further continuously subcultured and cultured. As a result, cells of subculture 17 (i.e., cells subjected to 17 times of subcultures) could be obtained. From these results, it is found that the cells obtained after completion of the freezing-thawing treatment can be subcultured at least 17 times.

Using a senescence marker, senescence associated β-galactosidase (SA-β-Gal), as an indicator, amniotic MSCs were subjected to a cellular senescence assay (cell staining). As a result, it was found that amniotic MSCs having a relatively large sizes (i.e., amniotic MSCs having a low specific growth rate, namely, amniotic MSCs having relatively low proliferative ability) were SA-β-Gal-positive senescent cells (Fig. 5), whereas amniotic MSCs having a relatively small size (i.e., amniotic MSCs having a high specific growth rate, namely, amniotic MSCs having relatively high proliferative ability) were SA-β-Gal-negative non-senescent cells.

As a result of performing flow cytometry, it was found that the cells of main culture 1 and the cells of subculture 1 were both positive for CD90, CD105 and CD73, and were both negative for CD106, CD324, CD326, CD45, CD34 and HLA-DR. The rate of CD106-positive cells in a cell population comprising the cells of main culture 1 was 1.4%. On the other hand, the rate of CD106-positive cells in a cell population comprising the cells of subculture 1 was 0.0%. These results demonstrate that the cells of main culture 1 and the cells of subculture 1 are amniotic mesenchymal stem cells, which hardly comprise epithelial cells, hematopoietic cells, and vascular endothelial cells. The obtained amniotic mesenchymal stem cells were viable cells preferable for the production of cell formulations.

(Example 2)

Amniotic MSCs having a high specific growth rate (amniotic MSCs having relatively high proliferative ability) were obtained by a series of Step 2-1 to Step 2-8, as described below.

(Step 2-1: Collection of amnion)

An fetal membrane and a placenta were aseptically collected as fetal appendages from a pregnant woman with an elective Caesarean section case, after the obtaining of informed consent. The obtained fetal membrane and placenta were placed in a sterilized tray filled with a normal saline, and amnion was then manually peeled from the edge of the fetal membrane. The amnion was washed twice with a Hanks' balanced salt solution (free of Ca and Mg), and adhered blood and clots were then removed from the amnion.

(Step 2-2: Enzyme treatment of amnion and recovery of amniotic MSCs)

5 mL of a Hanks' balanced salt solution (containing Ca and Mg) comprising 300 CDU/mL purified collagenase and 200 PU/mL dispase I was added per 1 g of amnion, and the obtained mixture was then shaken and stirred under conditions of 37°C, 90 minutes and 60 rpm. An enzymatically treated product of the amnion was suspended in two times its volume of αMEM supplemented with 10% FBS. The obtained cell suspension was filtered through a nylon mesh filter (pore size: 100 μm), and a filtrate containing amniotic MSCs was then recovered. The obtained filtrate was centrifuged at 400 x g for 5 minutes to remove a supernatant therefrom. The obtained cell pellets were suspended in αMEM supplemented with 10% FBS to obtain a cell suspension.

(Step 2-3: Pre-culture 1)

The cell population obtained from the enzymatically treated product of the amnion was seeded on a 1890-$cm^2$ (total culture surface area) plastic culture vessel, and was then cultured in αMEM supplemented with 10% FBS, until the confluent percentage became 90% or more and 95% or less.

(Step 2-4: Pre-culture 2)

The adhered cells obtained in Step 2-3 were treated with EDTA-trypsin, so that the cells were peeled from the plastic culture vessel. The obtained cell suspension was centrifuged to remove a supernatant therefrom. The obtained cell pellets were suspended in αMEM supplemented with 10% FBS. A total amount of the obtained cell suspension was seeded on a 6300-$cm^2$ (total culture surface area) plastic culture vessel, and was then cultured in αMEM supplemented with 10% FBS, until the confluent percentage became 90% or more and 95% or less.

(Step 2-5: Pre-culture 3)

The adhered cells obtained in Step 2-4 were treated with EDTA-trypsin, so that the cells were peeled from the plastic culture vessel. The obtained cell suspension was centrifuged to remove a supernatant therefrom. The obtained cell pellets were suspended in αMEM supplemented with 10% FBS. A total amount of the obtained cell suspension was seeded on a 2.5-$m^2$ (total culture surface area) plastic culture vessel, and was then cultured in αMEM supplemented with 10% FBS, until the confluent percentage became 90% or more and 95% or less.

(Step 2-6: Stimulation treatment of amniotic MSCs by freezing-thawing)

The adhered cells obtained in Step 2-5 were treated with EDTA-trypsin, so that the cells were peeled from the plastic culture vessel. The obtained cell suspension was centrifuged to remove a supernatant therefrom. The obtained cell pellets were suspended in an RPMI1640 medium supplemented with 0.5% (w/v) human serum albumin (HSA), to a concentration of 2.0 x $10^7$ cells/mL or less. A cryopreservation solution comprising 12% (w/v) HES, 10% (w/v) DMSO, and 8% (w/v) HSA was prepared. The cell suspension was mixed with an equal amount of the cryopreservation solution on ice, and the obtained mixture was then dispensed into four freezing bags. Using a program freezer, each freezing bag was cooled to -40°C at a freezing rate of -2°C/min or more and -1°C/min or less, and was then cooled to -90°C at a freezing rate of -10°C/min. Thereafter, each freezing bag was cryopreserved at -150°C in an ultra-low temperature freezer for 14 days. One of the thus cryopreserved four freezing bags was immersed in a thermostatic bath at 37°C, so that it was promptly melted.

(Step 2-7: Main culture 1)

(Method)

The melted cell suspension obtained in Step 2-6 (corresponding to a single freezing bag) was centrifuged to remove a supernatant therefrom. The obtained cell pellets were suspended in αMEM supplemented with 10% FBS. A total amount of the obtained cell suspension was seeded on a 2.5-$m^2$ (total culture surface area) plastic culture vessel, and was then cultured in αMEM supplemented with 10% FBS, until the confluent percentage became 90% or more and 95% or less. The cultured cells were subjected to an EDTA-trypsin treatment and centrifugation, to recover cells (a cell population comprising amniotic MSCs having a high specific growth rate, namely, a cell population comprising amniotic MSCs having relatively high proliferative ability).

(Results)

The number of cells obtained in Step 2-7 was 7.6 x $10^8$ cells. In addition, in the present step, the number of days, which was required for culturing cells after seeding thereof until the cells have reached a confluent percentage of 90% or more and 95% or less, was 10 days. From these results, it is found that 3.0 x $10^3$ (cells/$cm^2$/day) cells were obtained per batch culture. The specific growth rate of the obtained cells was 0.14 (1/day). Moreover, the obtained amniotic MSCs were viable cells preferable for the production of cell formulations. The aforementioned results demonstrate that amniotic MSCs necessary for the production of cell formulations can be promptly prepared and/or produced in large amounts.

(Step 2-8: Main culture 2)

(Method)

A total amount of the cells obtained in Step 2-7 was seeded on a 10.1-$m^2$ (total culture surface area) plastic culture vessel, and was then cultured in αMEM supplemented with 10% FBS, until the confluent percentage became 90% or more and 95% or less. The cultured cells were subjected to an EDTA-trypsin treatment and centrifugation, to recover cells (a cell population comprising amniotic MSCs having a high specific growth rate, namely, a cell population comprising amniotic MSCs having relatively high proliferative ability).

(Results)

The number of cells obtained in Step 2-8 was 3.0 x $10^9$ cells. In addition, in the present step, the number of days, which was required for culturing cells after seeding thereof until the cells have reached a confluent percentage of 90% or more and 95% or less, was 9 days. From these results, it is found that 3.3 x $10^3$ (cells/$cm^2$/day) cells were obtained per batch culture. The specific growth rate of the obtained cells was 0.15 (1/day). Moreover, the obtained amniotic MSCs were viable cells preferable for the production of cell formulations. The aforementioned results demonstrate that amniotic MSCs necessary for the production of cell formulations can be promptly prepared and/or produced in large amounts.

(Example 3)

Amniotic MSCs having a high specific growth rate (amniotic MSCs having relatively high proliferative ability) were obtained by a series of Step 3-1 to Step 3-9, as described below.

(Step 3-1: Collection of amnion)

An fetal membrane and a placenta were aseptically collected as fetal appendages from a pregnant woman with an elective Caesarean section case, after the obtaining of informed consent. The obtained fetal membrane and placenta were placed in a sterilized tray filled with a normal saline, and amnion was then manually peeled from the edge of the fetal membrane. The amnion was washed twice with a Hanks' balanced salt solution (free of Ca and Mg), and adhered blood and clots were then removed from the amnion.

(Step 3-2: Enzyme treatment of amnion and recovery of amniotic MSCs)

(Method)

5 mL of a Hanks' balanced salt solution (containing Ca and Mg) comprising 300 CDU/mL purified collagenase and 200 PU/mL dispase I was added per 1 g of amnion, and the obtained mixture was then shaken and stirred under conditions of 37°C, 90 minutes and 60 rpm. An enzymatically treated product of the amnion was suspended in two times its volume of αMEM supplemented with 10% FBS. The obtained cell suspension was filtered through a nylon mesh filter (pore size: 100 μm), and a filtrate containing amniotic MSCs was then recovered. Undigested amnion remaining on the nylon mesh filter was evaluated by hematoxylin-eosin (HE) staining. The obtained filtrate was centrifuged at 400 x g for 5 minutes to remove a supernatant therefrom. The obtained cell pellets were suspended in αMEM supplemented with 10% FBS and 1 x Antibiotic-Antimycotic (manufactured by Thermo Fisher Scientific), to obtain a cell suspension. A Turk's staining solution was mixed with a portion of the obtained cell suspension, and a cell count was then measured. The amniotic MSCs recovered from the filtrate were stained with a labeled antibody at 4°C for 15 minutes, and propidium iodide or 7-AAD (7-Amino-Actinomycin D) was then added to the amniotic MSCs to remove dead cells. Thereafter, a surface antigen was analyzed using a flow cytometer (FACS Canto: Becton, Dickinson and Company, BD Japan (hereinafter abbreviated as "BD"). An anti-CD90-FITC antibody (BD) was used herein as a labeled antibody.

(Results)

As a result of the hematoxylin-eosin (HE) staining, it was found that an epithelial cell layer was undigested and maintained in the amnion remaining on the filter, and that large quantities of epithelial cells were present in the epithelial cell layer. Moreover, an extracellular matrix layer was digested in the amnion remaining on the filter, and there were found no amniotic MSCs on the filter. These results demonstrate that a filtrate containing almost no epithelial cells was recovered by adopting collagenase and dispase, which target the extracellular matrix layer of the amnion and do not target the epithelial cell layer thereof.

As a result of the measurement of a cell count by Turk's staining, it was confirmed that the cell count obtained from amniotic tissues was 1.5 x $10^6$ cells per g of tissues.

As a result of the flow cytometry, a cell population comprising the cells recovered from the filtrate was positive for CD90. These results demonstrate that the cells recovered from the filtrate were mesenchymal stem cells.

(Step 3-3: Pre-culture 1)

A portion of the cell population obtained from the enzymatically treated product of the amnion was seeded on a 150-$cm^2$ plastic culture vessel, and was then cultured in $\alpha$MEM supplemented with 10% FBS and 1 x Antibiotic-Antimycotic for 3 days.

(Step 3-4: Pre-culture 2)

The adhered cells obtained in Step 3-3 were treated with TrypLE Select (1x) (manufactured by Thermo Fisher Scientific), so that the cells were peeled from the plastic culture vessel. The obtained cell suspension was centrifuged to remove a supernatant therefrom. The obtained cell pellets were suspended in $\alpha$MEM supplemented with 10% FBS and 1 x Antibiotic-Antimycotic. A portion of the obtained cell suspension was seeded on a 150-$cm^2$ plastic culture vessel, and was then cultured in $\alpha$MEM supplemented with 10% FBS and 1 x Antibiotic-Antimycotic for 7 days.

(Step 3-5: Pre-culture 3)

(Method)

The adhered cells obtained in Step 3-4 were treated with TrypLE Select (1x) (manufactured by Thermo Fisher Scientific), so that the cells were peeled from the plastic culture vessel. The obtained cell suspension was centrifuged to remove a supernatant therefrom. The obtained cell pellets were suspended in $\alpha$MEM supplemented with 10% FBS and 1 x Antibiotic-Antimycotic. A portion of the obtained cell suspension was seeded on a 150-$cm^2$ plastic culture vessel, and was then cultured in $\alpha$MEM supplemented with 10% FBS and 1 x Antibiotic-Antimycotic for 8 days. The cells obtained by this pre-culture are referred to as cells of pre-culture 3. The cells of pre-culture 3 were used in the subsequent tests.

(Results)

After completion of the culture for 8 days, the cells of pre-culture 3 were obtained at a cell density of 6.7 x $10^3$ cells/$cm^2$. In this case, the specific growth rate of the cells of pre-culture 3 was 0.11 (1/day).

(Step 3-6: Stimulation treatment of amniotic MSCs by freezing-thawing)

The adhered cells obtained in Step 3-5 (the cells of pre-culture 3) were treated with TrypLE Select (1x), so that the cells were peeled from the plastic culture vessel. The obtained cell suspension was centrifuged to remove a supernatant therefrom. The obtained cell pellets were suspended in an RPMI1640 medium supplemented with 0.5% (w/v) human serum albumin (HSA), to a concentration of 2.0 x $10^6$ cells/mL. A cryopreservation solution comprising 12% (w/v) HES, 10% (w/v) DMSO, and 8% (w/v) HSA was prepared. The cell suspension was mixed with an equal amount of the cryopreservation solution on ice, and the obtained mixture was then dispensed into a cryotube. Using a program freezer, the cryotube was cooled to -40°C at a freezing rate of -2°C/min or more and -1°C/min or less, and was then cooled to -90°C at a freezing rate of -10°C/min. Thereafter, the cryotube was cryopreserved at -150°C in an ultra-low temperature freezer for 30 days. Thereafter, the cryopreserved cryotube was immersed in a thermostatic bath at 37°C, so that it was promptly melted. A portion of the melted cell suspension was collected, and was then stained with trypan blue. After that, a cell count, a survival rate, and a size were measured using a hemocytometer.

(Results)

The diameter of the amniotic MSCs in a floating state, immediately after completion of the freezing-thawing treatment, was 22.2 ± 5.0 μm (i.e., average diameter: 22.2 μm).

(Step 3-7: Main culture 1)

(Method)

The melted cell suspension obtained in Step 3-6 was centrifuged to remove a supernatant therefrom. The obtained cell pellets were suspended in αMEM supplemented with 10% FBS and 1 x Antibiotic-Antimycotic. A portion of the obtained cell suspension was seeded on a 150-$cm^2$ plastic culture vessel, and was then cultured in αMEM supplemented with 10% FBS and 1 x Antibiotic-Antimycotic for 7 days. Thereafter, the cultured cells were subjected to a TrypLE Select (1x) treatment and centrifugation, to recover cells (a cell population comprising amniotic MSCs having a high specific growth rate, namely, a cell population comprising amniotic MSCs having relatively high proliferative ability). The cells obtained by this step are referred to as cells of main culture 1.

(Results)

The number of the cells of main culture 1 obtained in Step 3-7 was 2.2 x $10^6$ cells. From this result, it is found that 2.1 x $10^3$ (cells/$cm^2$/day) cells were obtained per batch culture. The specific growth rate of the obtained cells was 0.21 (1/day). Moreover, the obtained amniotic MSCs were viable cells preferable for the production of cell formulations. The aforementioned results demonstrate that amniotic MSCs necessary for the production of cell formulations can be promptly prepared and/or produced in large amounts.

(Step 3-8: Main culture 2)

(Method)

A portion of the cells obtained in Step 3-7 was seeded at a seeding density of 6.0 x $10^3$ cells/$cm^2$ on a 150-$cm^2$ plastic culture vessel, and was then cultured in αMEM supplemented with 10% FBS and 1 x Antibiotic-Antimycotic for 5 days. Thereafter, the cultured cells were subjected to a TrypLE Select (1x) treatment and centrifugation, to recover cells (a cell population comprising amniotic MSCs having a high specific growth rate, namely, a cell population comprising amniotic MSCs having relatively high proliferative ability). The cells obtained by this step are referred to as cells of subculture 1.

(Results)

The number of the cells of subculture 1 obtained in Step 3-8 was 4.6 x $10^6$ cells. From this result, it is found that 4.4 x $10^3$ (cells/$cm^2$/day) cells were obtained per batch culture. The specific growth rate of the obtained cells was 0.32 (1/day). Moreover, the obtained amniotic MSCs were viable cells preferable for the production of cell formulations. The aforementioned results demonstrate that amniotic MSCs necessary for the production of cell formulations can be promptly prepared and/or produced in large amounts.

(Step 3-9: Main culture 3)

(Method)

A portion of the cells of subculture 1 obtained in Step 3-8 was seeded at a seeding density of 2.5 x $10^3$ cells/$cm^2$ on a 150-$cm^2$ plastic culture vessel, and was then cultured in αMEM supplemented with 10% FBS and 1 x Antibiotic-Antimycotic for 6 days. Thereafter, the cultured cells were subjected to a TrypLE Select (1x) treatment and centrifugation, to recover cells (a cell population comprising amniotic MSCs having a high specific growth rate, namely, a cell population comprising amniotic MSCs having relatively high proliferative ability). The cells obtained by this step are referred to as cells of subculture 2.

Using Cellular Senescence Detection Kit (SA-β-Gal Staining) (Cell Biolabs), the cells of pre-culture 3 and the cells of subculture 2 were subjected to a cellular senescence assay (cell staining). In addition, a portion was collected from each of the cells of main culture 1, the cells of subculture 1 and the cells of subculture 2, it was then stained with a labeled antibody at 4°C for 15 minutes, and propidium iodide or 7-AAD (7-Amino-Actinomycin D) was then added to the cells to remove dead cells. Thereafter, a surface antigen was analyzed using a flow cytometer (FACS Canto: BD). An anti-CD90-FITC antibody (BD), an anti-CD 105-FITC antibody (Ancell), an anti-CD73-PE antibody (BD), an anti-CD 106-PE antibody (Miltenyi Biotec), an anti-CD324-PE antibody (BD), an anti-CD326-FITC antibody (BD), an anti-CD45-FITC antibody (BD), an anti-CD34-PE antibody (BD) and an anti-HLA-DR-FITC antibody (BD) were used herein as labeled antibodies described above. The rate of mesenchymal stem cells positive for specific expression markers (CD106 and CD90) in the cell population was calculated by the following procedures (1) to (3):

(1) The measurement results were developed on a histogram, in which the longitudinal axis indicated cell count, and the horizontal axis indicated the fluorescence intensity of a pigment used to label an antibody.
(2) Fluorescence intensity, at which a cell population having stronger fluorescence intensity accounted for 0.1% to 1.0% in all cells measured with an isotype control antibody, was determined.
(3) The rate of cells having fluorescence intensity higher than the fluorescence intensity determined in the above (2) in all cells measured by antibodies reacting against the specific expression markers (CD106 and CD90) was calculated.

(Results)

The number of the cells of subculture 2 obtained in Step 3-9 was 3.9 x $10^6$ cells. From this result, it is found that 3.7 x $10^3$ (cells/$cm^2$/day) cells were obtained per batch culture. The specific growth rate of the obtained cells was 0.39 (1/day). Moreover, the obtained amniotic MSCs were viable cells preferable for the production of cell formulations. The aforementioned results demonstrate that amniotic MSCs necessary for the production of cell formulations can be promptly prepared and/or produced in large amounts.

In order to examine the possible subculture number, the cells of subculture 2 obtained in Step 3-9 were further continuously subcultured and cultured. As a result, cells of subculture 10 (i.e., cells subjected to 10 times of subcultures) could be obtained. From these results, it is found that the cells obtained after completion of the freezing-thawing treatment can be subcultured at least 10 times.

Using a senescence marker, senescence associated β-galactosidase (SA-β-Gal), as an indicator, amniotic MSCs were subjected to a cellular senescence assay (cell staining). As a result, it was found that the cells of pre-culture 3 were positive for SA-β-Gal, whereas the cells of subculture 2 were negative for SA-β-Gal. The rate of SA-β-Gal-negative mesenchymal stem cells in a cell population comprising the cells of pre-culture 3 was 2.9 ± 2.3% (average rate: 2.9%). On the other hand, the rate of SA-β-Gal-negative mesenchymal stem cells in a cell population comprising the cells of subculture 2 was 94.3 ± 3.9% (average rate: 94.3%).

As a result of performing flow cytometry, it was found that the cells of main culture 1, the cells of subculture 1, and the cells of subculture 2 were all positive for CD90, CD105 and CD73, and were all negative for CD106, CD324, CD326, CD45, CD34 and HLA-DR. The rate of CD106-positive mesenchymal stem cells in a cell population comprising the cells of main culture 1, the cells of subculture 1 or the cells of subculture 2 was always 0.0%. On the other hand, the rate of CD90-positive mesenchymal stem cells in a cell population comprising the cells of main culture 1, the cells of subculture 1 or the cells of subculture 2 was 95%, 99%, and 97%, respectively. These results demonstrate that the cells of main culture 1, the cells of subculture 1 and the cells of subculture 2 are amniotic MSCs, which hardly comprise epithelial cells, hematopoietic cells, and vascular endothelial cells. The obtained amniotic MSCs were viable cells preferable for the production of cell formulations.

(Example 4)

Amniotic MSCs having different specific growth rates were compared and analyzed, in terms of gene expression, according to the following Step 4-1 to Step 4-3.

(Step 4-1: Extraction of total RNA)

(Method)

The cells of pre-culture 3 in an adhesion state obtained in Step 3-5 of Example 3 (specific growth rate: 0.11 (1/day)) and the cells of subculture 2 in an adhesion state obtained in Step 3-9 of Example 3 (specific growth rate: 0.39 (1/day)) were each peeled from a plastic culture vessel, using a cell scraper (manufactured by Corning), and were then recovered by centrifugation. RNAlater (manufactured by Thermo Fisher Scientific) was added to the obtained cell pellets to stably preserve RNA, and thereafter, total RNA was extracted and purified, using RNeasy Plus Mini kit (manufactured by QIAGEN).

(Results)

As a result of an electrophoretic pattern analysis using LabChip kit and Agilent 2100 Bioanalyzer, two clear peaks of ribosomal RNAs (18SrRNA and 28SrRNA) were detected in all RNA samples. Moreover, as a result of quantification using a spectrophotometer NanoDrop (manufactured by Thermo Fisher Scientific), the A260/A280 ratio was a value from 1.8 to 2.1 in all RNA samples. In view of the foregoing, the two RNA samples were each confirmed to have a purity suitable as an array analysis sample.

(Step 4-2: Obtaining of microarray data)

(Method)

cDNA was synthesized from 100 ng of total RNA by a reverse transcription reaction, and the synthesized cDNA was then transcribed into cRNA by *in vitro* transcription, followed by biotin labeling (using 3'IVT PLUS Reagent Kit). 10.0 μg of the labeled cRNA was added to a hybridization buffer, and hybridization was then carried out on Human GeneGenome U133A 2.0 Array (manufactured by Affymetrix) for 16 hours. Thereafter, the resultant was washed with GeneChip Fluidics Station 450 (manufactured by Affymetrix), was then stained with phycoerythrin, and was then scanned using GeneChip Scanner 3000 7G (manufactured by Affymetrix). Thereafter, image analysis was carried out using AGCC (Affymetrix GeneChip Command Console Software) (manufactured by Affymetrix), and the results were then numerized using Affymetrix Expression Console (manufactured by Affymetrix).

(Results)

In the data analyzed by the Affymetrix Expression Console, the signaling (3'/5') ratio of GAPDH as a housekeeping gene in each sample was 0.98 and 1.04 (which were no more than 3 as an ideal value), respectively. Thus, it was confirmed that the obtained cRNA had no problems with the quality.

(Step 4-3: Analysis of microarray data)

(Method)

Numerized data files from two arrays were compared and analyzed using the analysis software GeneSpring GX (manufactured by Agilent Technologies). The normalized value of a sample of the cells of subculture 2 was divided by the normalized value of a sample of the cells of pre-culture 3 used as a control, and probes having a quotient of 2 or more were selected, so as to search for a gene whose expression was fluctuated by 2 times or more (a gene having a Fold Change value of 2 times or more).

(Results)

The results obtained by comparing the cells of subculture 2 with the cells of pre-culture 3, in terms of the gene expression of metallothionein isoforms, are shown in Table 4.

[Table 4]

Table 4. Comparison of cells of subculture 2 with cells of pre-culture 3 in terms of expression of metallothionein family gene

| | | Metallothionein Isoforms | | | | | |
|---|---|---|---|---|---|---|---|
| | | MT1E | MT1F | MT1G | MT1H | MT1X | MT2A |
| Cells of pre-culture 3 | Fluorescence intensity | 2346.33 | 1865.68 | 1738.45 | 1574.86 | 4166.44 | 7890.23 |
| | Detection judgement | Detected | Detected | Detected | Detected | Detected | Detected |

(continued)

| | | Metallothionein Isoforms | | | | | |
|---|---|---|---|---|---|---|---|
| | | MT1E | MT1F | MT1G | MT1H | MT1X | MT2A |
| Cells of subculture 2 | Fluorescence intensity | 5014.80 | 4030.41 | 4343.23 | 6073.11 | 11298.00 | 17276.73 |
| | Detection judgement | Detected | Detected | Detected | Detected | Detected | Detected |
| Fold Change value (Expression variation value indicated by decimal system) | | 2.24 | 2.26 | 2.62 | 4.04 | 2.84 | 2.29 |

As shown in Table 4, it was found that, with regard to metallothionein-1E (MT1E), metallothionein-1F (MT1F), metallothionein-1G (MT1G), metallothionein-1H (MT1H), metallothionein-X (MT1X) and metallothionein-2A (MT2A), the cells of subculture 2 exhibited a Fold Change value that was 2 times or more than the cells of pre-culture 3. From these results, it became clear that the cells of subculture 2 highly expressed these metallothionein family genes.

(Example 5)

A composition comprising amniotic MSCs having a high specific growth rate was prepared, and the composition was cryopreserved and was then thawed. Thereafter, the survival rate of the cells comprised in the composition was measured.

(Method)

A composition consisting of 8.0 x $10^6$ cells of the amniotic MSCs (the cells of subculture 2) obtained in Step 3-9 of Example 3 and 1 mL of RPMI1640 medium supplemented with 20 mg of dextran, 52 mg of DMSO and 40 mg of human serum albumin was prepared. The composition was enclosed in a cryotube, and it was then preserved in a frozen state at -150°C for 7 days. Thereafter, the cryopreserved composition was promptly thawed in a thermostatic bath at 37°C, and was then left at rest at 25°C (room temperature) or 37°C (body temperature) for 1 or 2 hours. Thereafter, a portion of the obtained composition was collected, and was then stained with trypan blue. Subsequently, the survival rate of the cells was measured using a hemocytometer.

(Results)

The survival rate of the cells comprised in the composition immediately after thawing was 96.3 $\pm$ 1.2%. The survival rate of the cells comprised in the composition after leaving at rest at 25°C for 1 or 2 hours was 94.7 $\pm$ 1.5% and 93.0 $\pm$ 1.0%, respectively. The survival rate of the cells comprised in the composition after leaving at rest at 37°C for 1 or 2 hours was 92.7 $\pm$ 3.1% and 90.3 $\pm$ 2.9%, respectively. From these results, it was confirmed that a high cell survival rate of 90% or more can be maintained, after the cells have been left at rest for at least 2 hours, in both cases of 25°C and 37°C.

(Example 6)

(Production of cell formulations and administration)

A portion of the amniotic MSCs obtained in Step 2-8 of Example 2 or Step 3-9 of Example 3 (amniotic MSCs having a high specific growth rate, namely, amniotic MSCs having relatively high proliferative ability) was used to prepare a pharmaceutical composition. A pharmaceutical composition (cell formulation) consisting of 2.3 x $10^8$ amniotic MSCs having relatively high proliferative ability, and 25 mL of RPMI1640 medium supplemented with 0.50 g of HES or dextran, 1.3 g of DMSO and 1.0 g of human serum albumin was prepared. The pharmaceutical composition was enclosed in a freezing bag, and was then preserved in a frozen state.

Upon the use of the pharmaceutical composition, first, the cryopreserved pharmaceutical composition was promptly thawed at 37°C, and the thawed pharmaceutical composition was then diluted with a normal saline. Subsequently, the pharmaceutical composition could be administered to a patient by intravenous injection, intravenous drip infusion, or direct localized injection, while the composition was gently blended so that the cells in the diluted composition could be uniformly dispersed.

## Claims

**1.** A method for producing a cell population comprising mesenchymal stem cells, comprising:

treating a cell population comprising mesenchymal stem cells having different proliferative ability by physical stimulation or chemical stimulation, so as to select mesenchymal stem cells having relatively high proliferative ability, wherein

the selected mesenchymal stem cells having relatively high proliferative ability are negative for CD106, and the expression level of a metallothionein family gene is increased in comparison to the expression level thereof in the cell population before the treatment by the physical stimulation or chemical stimulation.

**2.** The method for producing a cell population according to claim 1, wherein the treatment of the cell population comprising mesenchymal stem cells having different proliferative ability by physical stimulation is freezing and subsequent thawing of the cell population.

**3.** The method for producing a cell population according to claim 1 or 2, wherein the treatment of the cell population comprising mesenchymal stem cells having different proliferative ability by physical stimulation comprises suspending the cell population in a cryopreservation solution, then freezing the obtained suspension, then maintaining the frozen state, and then thawing it.

**4.** The method for producing a cell population according to claim 3, wherein the frozen state is maintained for 2 days or more.

**5.** The method for producing a cell population according to any one of claims 1 to 4, which further comprises culturing and/or sub-culturing the cell population comprising the mesenchymal stem cells obtained in the selection step.

**6.** The method for producing a cell population according to any one of claims 1 to 5, which further comprises a cell population-obtaining step of subjecting a fetal appendage to an enzyme treatment, so as to obtain a cell population comprising mesenchymal stem cells having different proliferative ability.

**7.** The method for producing a cell population according to claim 6, which further comprises culturing the cell population obtained in the cell population-obtaining step.

**8.** The method for producing a cell population according to any one of claims 1 to 7, wherein the metallothionein family gene is at least one selected from the group consisting of MT1E, MT1F, MT1G, MT1H, MT1X, and MT2A.

**9.** The method for producing a cell population according to any one of claims 1 to 8, wherein the selected mesenchymal stem cells having relatively high proliferative ability are negative for SA-β-Gal.

**10.** The method for producing a cell population according to claim 9, wherein the average rate of the SA-β-Gal-negative mesenchymal stem cells in the cell population is 90% or more.

**11.** The method for producing a cell population according to any one of claims 1 to 10, wherein the selected mesenchymal stem cells having relatively high proliferative ability is positive for CD105, CD73, and CD90, and negative for CD45, CD34, CD11b, CD79alpha, and HLA-DR.

**12.** The method for producing a cell population according to any one of claims 1 to 11, wherein the specific growth rate of the selected mesenchymal stem cells having relatively high proliferative ability is 0.14 (1/day) or more.

**13.** The method for producing a cell population according to any one of claims 1 to 11, wherein the average diameter of the selected mesenchymal stem cells having relatively high proliferative ability that are in a floating state is 80% or less of the average diameter of mesenchymal stem cells having relatively low proliferative ability that are in a floating state.

**14.** Mesenchymal stem cells, which are negative for CD106, and wherein the expression level of a metallothionein family gene is increased in comparison to the expression level thereof in the cells before the treatment by the physical stimulation or chemical stimulation.

**15.** The mesenchymal stem cells according to claim 14, wherein the metallothionein family gene is at least one selected from the group consisting of MT1E, MT1F, MT1G, MT1H, MT1X, and MT2A.

**16.** The mesenchymal stem cells according to claim 14 or 15, wherein the mesenchymal stem cells are negative for SA-β-Gal.

**17.** The mesenchymal stem cells according to any one of claims 14 to 16, wherein the mesenchymal stem cells are positive for CD105, CD73, and CD90, and negative for CD45, CD34, CD11b, CD79alpha, and HLA-DR.

**18.** The mesenchymal stem cells according to any one of claims 14 to 17, wherein the specific growth rate of the mesenchymal stem cells is 0.14 (1/day) or more.

**19.** The mesenchymal stem cells according to any one of claims 14 to 18, wherein the average diameter of the mesenchymal stem cells that are in a floating state is 80% or less of the average diameter of mesenchymal stem cells having relatively low proliferative ability that are in a floating state.

**20.** A cell population comprising mesenchymal stem cells, wherein
the expression level of a metallothionein family gene in the mesenchymal stem cells is increased in comparison to the expression level thereof in the cells before the treatment by the physical stimulation or chemical stimulation, and the mesenchymal stem cells are negative for CD106.

**21.** The cell population according to claim 20, wherein the rate of the CD 106-positive mesenchymal stem cells in the cell population is less than 5%.

**22.** The cell population according to claim 20 or 21, wherein the average rate of the SA-β-Gal-negative mesenchymal stem cells in the cell population is 90% or more.

**23.** A pharmaceutical composition comprising the mesenchymal stem cells according to any one of claims 14 to 19, or the cell population according to any one of claims 20 to 22, and a pharmaceutically acceptable medium.

**24.** The pharmaceutical composition according to claim 23, wherein the single dose of the mesenchymal stem cells to a human is $10^9$ cells/kg body weight or less.

**25.** The pharmaceutical composition according to claim 23 or claim 24, which is a therapeutic agent for a disease selected from immune-related disease, graft-versus-host disease, inflammatory bowel disease, systemic lupus erythematosus, connective tissue disease, radiation enteritis, hepatic cirrhosis, stroke, or atopic dermatitis.

[Fig.1]

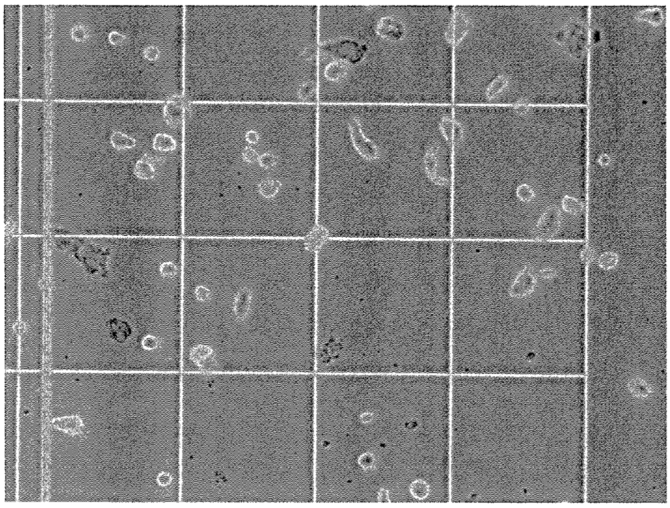

[Fig.2]

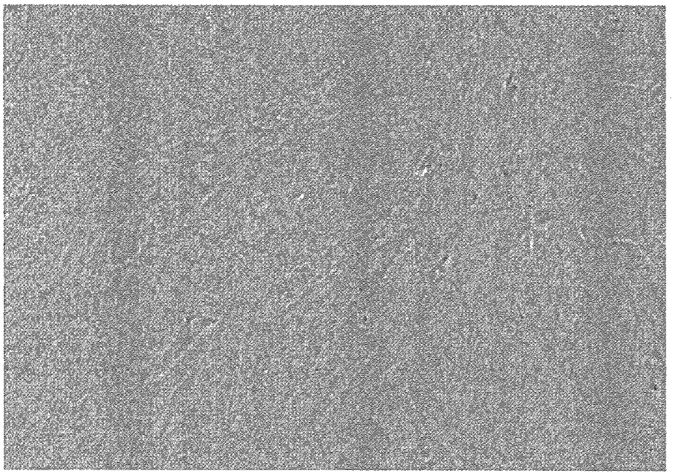

[Fig.3]

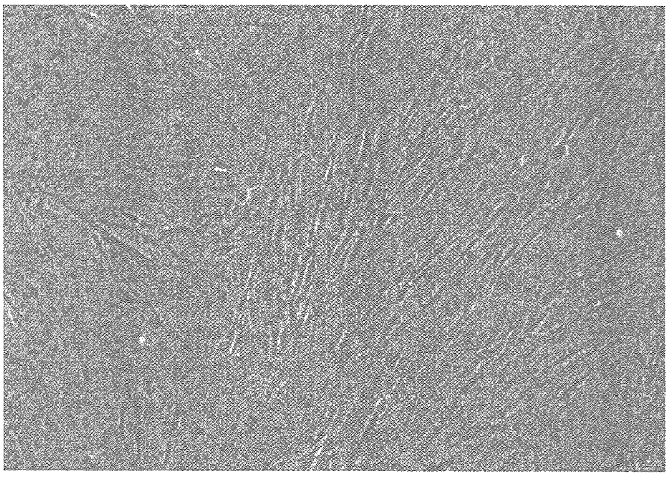

[Fig.4]

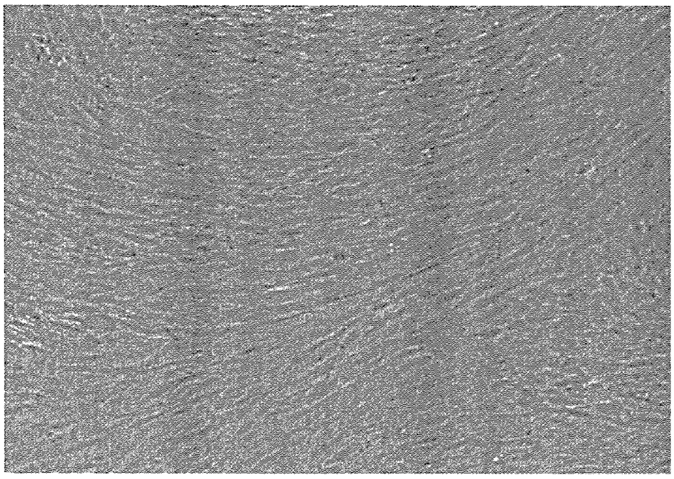

[Fig.5]

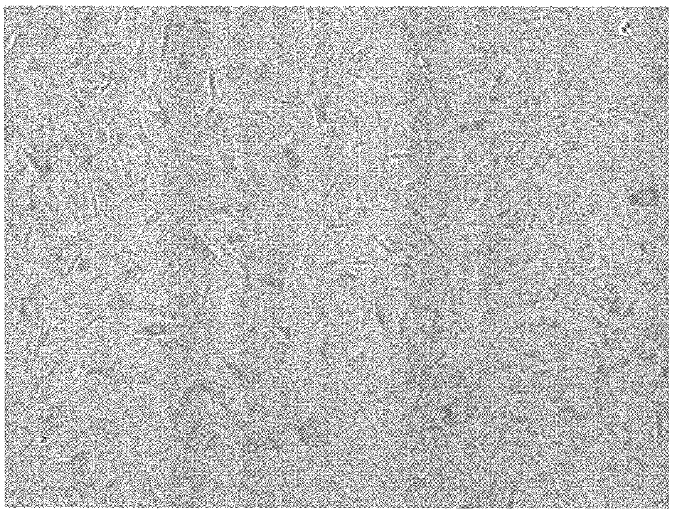

[Fig.6]

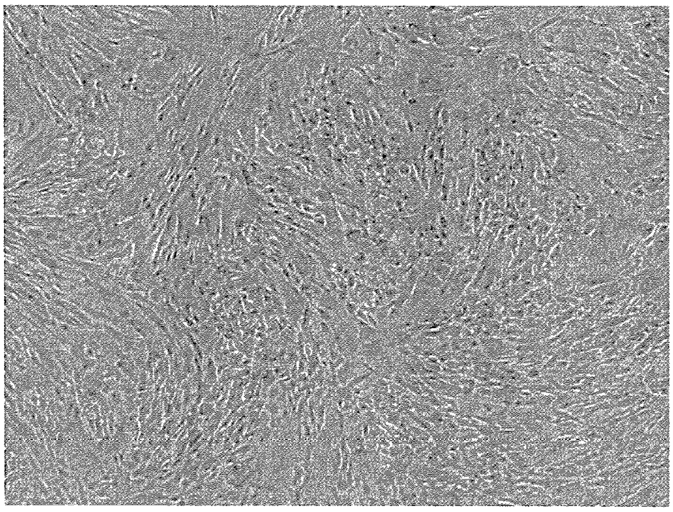

[Fig.7]

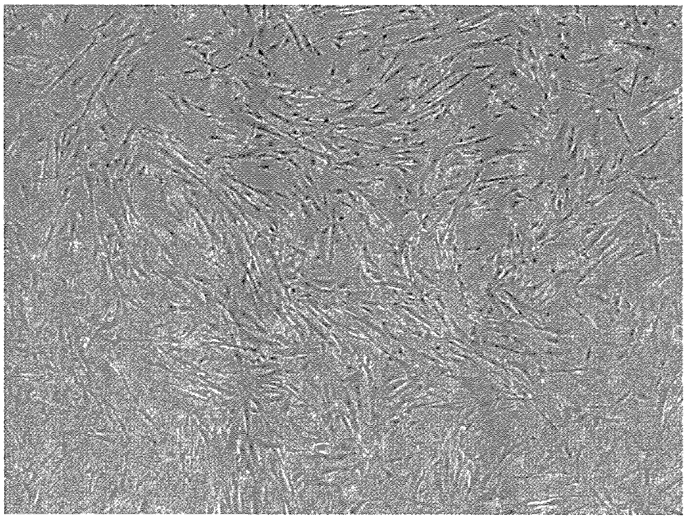

**INTERNATIONAL SEARCH REPORT**

International application No.
PCT/JP2016/081860

A. CLASSIFICATION OF SUBJECT MATTER
*C12N5/0775*(2010.01)i, *A61K35/28*(2015.01)i, *A61P1/04*(2006.01)i, *A61P1/16*(2006.01)i, *A61P9/10*(2006.01)i, *A61P17/06*(2006.01)i, *A61P37/02*(2006.01)i

According to International Patent Classification (IPC) or to both national classification and IPC

B. FIELDS SEARCHED

Minimum documentation searched (classification system followed by classification symbols)
C12N5/0775, A61K35/28, A61P1/04, A61P1/16, A61P9/10, A61P17/06, A61P37/02

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2017 |
| Kokai Jitsuyo Shinan Koho | 1971-2017 | Toroku Jitsuyo Shinan Koho | 1994-2017 |

Electronic data base consulted during the international search (name of data base and, where practicable, search terms used)
CAplus/MEDLINE/EMBASE/BIOSIS/WPIDS(STN), JSTPlus/JMEDPlus/JST7580(JDreamIII)

C. DOCUMENTS CONSIDERED TO BE RELEVANT

| Category* | Citation of document, with indication, where appropriate, of the relevant passages | Relevant to claim No. |
|---|---|---|
| X | MOON J.H. et al., Successful vitrification of human amnion-derived mesenchymal stem cells. Hum. Reprod., 2008, vol.23, no.8, pp.1760-1770 (BACKGROUND, Figure 4, Table I, III) | 1-25 |
| A | WO 2015/025810 A1 (National Cerebral and Cardiovascular Center), 26 February 2015 (26.02.2015), claims & US 2016/0228474 A1 claims & JP 2015-61520 A & EP 3037523 A1 | 1-25 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 10 January 2017 (10.01.17) | 24 January 2017 (24.01.17) |

| Name and mailing address of the ISA/ | Authorized officer |
|---|---|
| Japan Patent Office<br>3-4-3,Kasumigaseki,Chiyoda-ku,<br>Tokyo 100-8915,Japan | Telephone No. |

Form PCT/ISA/210 (second sheet) (January 2015)

**INTERNATIONAL SEARCH REPORT**

International application No.
PCT/JP2016/081860

C (Continuation). DOCUMENTS CONSIDERED TO BE RELEVANT

| Category* | Citation of document, with indication, where appropriate, of the relevant passages | Relevant to claim No. |
|---|---|---|
| A | WO 2015/034212 A1 (MEDIPOST CO., LTD.), 12 March 2015 (12.03.2015), claims & US 2016/0222353 A1 claims & JP 2016-534736 A & EP 3041930 A1 | 1-25 |
| A | JP 2014-501110 A (The Administrators of the Tulane Educational Fund), 20 January 2014 (20.01.2014), claims & WO 2012/088225 A2 claims & EP 2655602 A2 & US 2013/0273570 A1 | 1-25 |
| A | JP 2008-220334 A (Kyoto University), 25 September 2008 (25.09.2008), claims (Family: none) | 1-25 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2015061520 A
- JP 2015015963 A
- JP 2014501110 A
- WO 2013077428 A
- JP 2012509087 A
- JP 2014501249 A
- JP 2013256515 A
- JP 2014185173 A
- JP 2015038059 A
- JP 2015110659 A
- JP 2006521121 A
- JP 2009542727 A
- JP 2010535715 A
- JP 2014224117 A
- JP 2015061862 A
- JP 2002511094 A
- JP 2004507454 A
- JP 2010505764 A
- JP 2011514901 A
- JP 2013064003 A
- JP 2015131795 A
- JP 2010518096 A
- JP 2015178498 A
- JP 5950577 A


### Non-patent literature cited in the description


- **ONKEN JE et al.** *American College of Gastroenterology Conference,* 2006
- **GARCIA-OLMO D et al.** *Dis Colon Rectum,* 2005, vol. 48, 1416-23
- *J Am Coll Cardiol.,* 08 December 2009, vol. 54 (24), 2277-2286
- *Brain,* 2011, vol. 134, 1790-1807
- *Ann Thorac Surg,* 2013, vol. 95, 1827-33

SEQUENCE LISTING

```
<110>  Kaneka Corporation
<110>  National Cerebral and Cardiovascular Center
<120>  METHOD FOR PRODUCING CELL POPULATION COMPRISING MESENCHYMAL STEM
CELLS, MESENCHYMAL STEM CELL, CELL POPULATION, AND PHARMACEUTICAL COMPOSITION
<130>  161494A
<160>  12
<170>  PatentIn version 3.5
<210>  1
<211>  186
<212>  DNA
<213>  Human
<400>  1
atggacccca actgctcttg cgccactggt ggctcctgca cgtgcgccgg ctcctgcaag       60
tgcaaagagt gcaaatgcac ctcctgcaag aagagctgct gttcctgctg ccccgtgggc      120
tgtgccaagt gtgcccaggg ctgcgtctgc aaaggggcat cggagaagtg cagctgctgt      180
gcctga                                                                 186
<210>  2
<211>  186
<212>  DNA
<213>  Human
<400>  2
atggacccca actgctcctg cgccgctggt gtctcctgca cctgcgctgg ttcctgcaag       60
tgcaaagagt gcaaatgcac ctcctgcaag aagagctgct gctcctgctg ccccgtgggc      120
tgtagcaagt gtgcccaggg ctgtgtttgc aaaggggcgt cagagaagtg cagctgctgc      180
gactga                                                                 186
<210>  3
<211>  189
<212>  DNA
<213>  Human
<400>  3
atggacccca actgctcctg tgccgctgca ggtgtctcct gcacctgcgc cagctcctgc       60
aagtgcaaag agtgcaaatg cacctcctgc aagaagagct gctgctcctg ctgccctgtg      120
ggctgtgcca agtgtgccca gggctgcatc tgcaaagggg catcggagaa gtgcagctgc      180
tgcgcctga                                                              189
<210>  4
<211>  186
<212>  DNA
<213>  Human
<400>  4
atggacccca actgctcctg cgaggctggt ggctcctgcg cctgcgccgg ctcctgcaag       60
tgcaaaaagt gcaaatgcac ctcctgcaag aagagctgct gctcctgttg cccccctgggc     120
tgtgccaagt gtgcccaggg ctgcatctgc aaaggggcgt cagagaagtg cagctgctgt      180
gcctga                                                                 186
<210>  5
<211>  186
<212>  DNA
<213>  Human
<400>  5
atggacccca actgctcctg ctcgcctgtt ggctcctgtg cctgtgccgg ctcctgcaaa       60
tgcaaagagt gcaaatgcac ctcctgcaag aagagctgct gctcctgctg ccctgtgggc      120
tgtgccaagt gtgcccaggg ctgcatctgc aaagggacgt cagacaagtg cagctgctgt      180
gcctga                                                                 186
<210>  6
<211>  186
<212>  DNA
<213>  Human
<400>  6
atggatccca actgctcctg cgccgccggt gactcctgca cctgcgccgg ctcctgcaaa       60
tgcaaagagt gcaaatgcac ctcctgcaag aaaagctgct gctcctgctg ccctgtgggc      120
tgtgccaagt gtgcccaggg ctgcatctgc aaaggggcgt cggacaagtg cagctgctgc      180
gcctga                                                                 186
<210>  7
```

<211> 61
<212> PRT
<213> Human
<400> 7

```
Met Asp Pro Asn Cys Ser Cys Ala Thr Gly Gly Ser Cys Thr Cys Ala
1               5                   10                  15
Gly Ser Cys Lys Cys Lys Glu Cys Lys Cys Thr Ser Cys Lys Lys Ser
            20                  25                  30
Cys Cys Ser Cys Cys Pro Val Gly Cys Ala Lys Cys Ala Gln Gly Cys
        35                  40                  45
Val Cys Lys Gly Ala Ser Glu Lys Cys Ser Cys Cys Ala
    50                  55                  60
```

<210> 8
<211> 61
<212> PRT
<213> Human
<400> 8

```
Met Asp Pro Asn Cys Ser Cys Ala Ala Gly Val Ser Cys Thr Cys Ala
1               5                   10                  15
Gly Ser Cys Lys Cys Lys Glu Cys Lys Cys Thr Ser Cys Lys Lys Ser
            20                  25                  30
Cys Cys Ser Cys Cys Pro Val Gly Cys Ser Lys Cys Ala Gln Gly Cys
        35                  40                  45
Val Cys Lys Gly Ala Ser Glu Lys Cys Ser Cys Cys Asp
    50                  55                  60
```

<210> 9
<211> 62
<212> PRT
<213> Human
<400> 9

```
Met Asp Pro Asn Cys Ser Cys Ala Ala Ala Gly Val Ser Cys Thr Cys
1               5                   10                  15
Ala Ser Ser Cys Lys Cys Lys Glu Cys Lys Cys Thr Ser Cys Lys Lys
            20                  25                  30
Ser Cys Cys Ser Cys Cys Pro Val Gly Cys Ala Lys Cys Ala Gln Gly
        35                  40                  45
Cys Ile Cys Lys Gly Ala Ser Glu Lys Cys Ser Cys Cys Ala
    50                  55                  60
```

<210> 10
<211> 61
<212> PRT
<213> Human
<400> 10

```
Met Asp Pro Asn Cys Ser Cys Glu Ala Gly Gly Ser Cys Ala Cys Ala
1               5                   10                  15
Gly Ser Cys Lys Cys Lys Lys Cys Lys Cys Thr Ser Cys Lys Lys Ser
            20                  25                  30
Cys Cys Ser Cys Cys Pro Leu Gly Cys Ala Lys Cys Ala Gln Gly Cys
        35                  40                  45
Ile Cys Lys Gly Ala Ser Glu Lys Cys Ser Cys Cys Ala
    50                  55                  60
```

<210> 11
<211> 61
<212> PRT
<213> Human
<400> 11

```
Met Asp Pro Asn Cys Ser Cys Ser Pro Val Gly Ser Cys Ala Cys Ala
1               5                   10                  15
Gly Ser Cys Lys Cys Lys Glu Cys Lys Cys Thr Ser Cys Lys Lys Ser
            20                  25                  30
Cys Cys Ser Cys Cys Pro Val Gly Cys Ala Lys Cys Ala Gln Gly Cys
        35                  40                  45
Ile Cys Lys Gly Thr Ser Asp Lys Cys Ser Cys Cys Ala
```

```
    50                  55                  60
<210>  12
<211>  61
<212>  PRT
<213>  Human
<400>  12
Met Asp Pro Asn Cys Ser Cys Ala Ala Gly Asp Ser Cys Thr Cys Ala
1               5                   10                  15
Gly Ser Cys Lys Cys Lys Glu Cys Lys Cys Thr Ser Cys Lys Lys Ser
            20                  25                  30
Cys Cys Ser Cys Cys Pro Val Gly Cys Ala Lys Cys Ala Gln Gly Cys
        35                  40                  45
Ile Cys Lys Gly Ala Ser Asp Lys Cys Ser Cys Cys Ala
    50                  55                  60
```